(19) Europäisches Patentamt
European Patent Office
Office européen des brevets

(11) EP 0 943 918 B1

(12) **EUROPEAN PATENT SPECIFICATION**

(45) Date of publication and mention of the grant of the patent:
**17.08.2005 Bulletin 2005/33**

(51) Int Cl.[7]: **G01N 33/533**, G01N 33/58, G01N 33/52

(21) Application number: **99110086.8**

(22) Date of filing: **30.05.1996**

(54) **Fluorescent labelling complexes with large Stokes' shifts formed by coupling together cyanine and other fluorochromes capable of resonance energy transfer**

Fluoreszierende Markierungskomplexe mit grossem Stokes-Shift die durch Verbindung von Cyaninen und anderen resonanzenergieübertragungsfähigen Fluorochromen zusammengestellt sind

Complexe avec un grand décalage de stokes pour marquage fluorescent formé par coplage de cyanine et autres fluorochromes capables de transfert d'énergie résonance

(84) Designated Contracting States:
**AT BE CH DE ES FI FR GB IT LI NL SE**

(30) Priority: **07.06.1995 US 476880**

(43) Date of publication of application:
**22.09.1999 Bulletin 1999/38**

(62) Document number(s) of the earlier application(s) in accordance with Art. 76 EPC:
**96303879.9 / 0 747 700**

(73) Proprietor: **CARNEGIE MELLON UNIVERSITY**
**Pittsburgh Pennsylvania 15213 (US)**

(72) Inventors:
- **Waggoner, Alan Stuart**
  **Pittsburgh, PA 15213 (US)**
- **Mujumdar, Swati Ratnaker**
  **Glenshaw, PA 15166 (US)**
- **Mujumdar, Ratnaker Balvant**
  **Placentia CA 92870 (US)**

(74) Representative: **Rollins, Anthony John**
**GE Healthcare Limited**
**Amersham Place**
**Little Chalfont, Bucks. HP7 9NA (GB)**

(56) References cited:
**EP-A- 0 609 894**
**EP-A- 0 710 668**
**WO-A-93/06482**
**WO-A-94/24213**
**US-A- 5 378 634**
**US-A- 5 453 505**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to fluorescent labelling complexes, and more particularly to low molecular weight fluorescent complexes with large Stokes' shifts and to their use in the preparation of fluorescent derivatives of target materials.

Fluorescence labelling is an important technology for detecting biological molecules. For example, antibodies can be labelled with fluorescent dyes. The binding of antibodies to their specific target molecules can then be monitored on the basis of a fluorescence signal, which may be detected with a spectrometer, immunofluorescence instrument, flow cytometer, or fluorescence microscope. In a similar way DNA sequences can be detected with fluorescence detection instruments after the DNA has been hybridized with a complementary DNA sequence that has been labelled with a fluorescent dye.

Energy transfer complexes containing covalently linked donor and acceptor molecules are known. For example, a model system was developed by Stryer and Haugland for the study of the dependence of singlet-singlet energy transfer on distance (Stryer, L. and Haugland, R.P., Proc.Nat.Acad.Sci., Vol.58, pp.720-26, (1967)). The synthesis and properties of new photochemical model compounds containing a cyanine dye and a porphyrin has been reported (Lindsey et al, Tetrahedron, Vol. 45, No.15, pp.4845-66, (1989)). Complexes containing fluorescent donor and acceptor chromophores have been described as substrates for the kinetic study and assay of hydrolytic enzymes (Carmel et al, FEBS Letters, Vol.30, No.1, p11, (1973)).

European Patent Application No.609894 discloses a labelling complex comprising a trinucleus dye represented by the general formula (1).

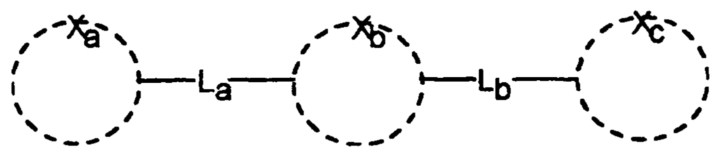

(1)

where Xa, Xb and Xc are independently substituted or unsubstituted heterocyclic rings containing one to three heteroatoms and La and Lb are conjugated methine chains. One of La and Lb may be omitted so as to link the heterocycles directly. The compounds of structure (1) can include a reactive group for forming a covalent linkage between the trinucleus dye and a biological substance. Compounds of such a formula are reported to have a large Stokes' shift (50-100nm). However, it is not thought that resonance energy transfer is involved in the process of fluorescence with those dyes.

European Patent Application No.601889 discloses nucleic acid probes containing a target binding sequence, a region of which is capable of forming one or more imperfect hairpins, and at least one donor label and at least one acceptor label covalently attached to the nucleotide sequence such that when one or more hairpin structures are formed, one of the donor and one of the acceptor moieties are in close proximity so as to allow resonance energy transfer between them. The labels are preferably fluorophores. Also disclosed is a method of using such probes for detecting a polynucleotide target such that when the probe interacts with a target sequence the distance between the fluorescent groups is caused to change, resulting in a change in the fluorescence emission of the probe.

Lee et al, Nucleic Acids Research, 20(10), 2471-2483, (1992) relates to fluorescent dye-labelled terminators for use in DNA sequencing. Disclosed is a ddG-bifluor dye consisting of a fluorescein and rhodamine dye covalently linked to each other and to a purine nucleotide. The absorption maxima of the dye complex are 498nm and 554nm and the emission maximum is 580nm.

Japanese Patent Application No. 05 60698 discloses single stranded DNA primers labelled with two kinds of fluorescent bodies in an energy transfer relationship for use in a sequencing method.

Multiparameter analysis using fluorescent labels with distinctly different emission wavelengths further increases the importance of this technology by providing a powerful tool for correlating multiple antigenic or genetic parameters in individual cells. In epifluorescence microscopy, a continuous light source with different sets of excitation and emission filters are used to excite and detect each fluorescent species. This approach works especially well if the absorption and emission wavelengths of each of the fluorophores are relatively close together (eg. Stokes' shifts of 15-30nm). Most of the highly fluorescent, low molecular weight fluorophores like the cyanines and xanthenes have narrow absorption and emission peaks and small Stokes' shifts. Up to 5 separate fluorescent labels have been analysed on the same specimen by microscopy using epifluorescence filter sets as described by DeBiasio et al, Journal of Cell Biology, Vol.105, pp.1613-1622, (1987).

While it is easy to find a single fluorophore that can be efficiently excited at a particular laser wavelength, it is difficult to find additional fluorescent labels with large enough Stokes' shifts to provide emission well separated from that of the first fluorophore. The naturally occurring phycobiliproteins are a class of multichromophore fluorescent photosystem proteins that have large wavelength shifts; see Oi, V.T., Glazer, A.N. and Stryer, L., Journal of Cell Biology, Vol.93, pp.981-986, (1982). These can be covalently coupled to antibodies and have been widely used in flow cytometry for 2-colour lymphocyte subset analysis. R-phycoerythrin (R-PE), a photosystem protein containing 34 bilin fluorophores which can be excited at 488nm with the widely available argon ion laser, has been especially useful. It fluoresces maximally at 575nm. R-PE and fluorescein can both be excited at 488nm, but R-PE can be readily discriminated with optical band pass interference filter sets from the fluorescein signal which appears at 525nm. Recently, 3-colour immunofluorescence by flow cytometry has become possible through the development of tandem conjugate labelling reagents that contain a reactive fluorescent dye which is excited at 488nm and fluoresces at 613nm, and is sold commercially under the name Duochrome, see: US Patent No.4876190. With another tandem fluorophore energy transfer from excited R-PE to the linked cyanine dye known as Cy-5 leads to fluorescence at 670nm (Waggoner et al. Ann. N. Y.Acad. Sci., Vol.677, pp.185-193, (1993)).

The phycobiliprotein-based labels are very fluorescent and provide excellent signals in 2- and 3-parameter experiments for detection of cell surface antigens. However these reagents have not been widely utilised for measurement of cytoplasmic antigens or for detection of chromosomal markers by fluorescence *in situ* hybridization because their large size (MW 210,000 Daltons) limits penetration into dense cell structures.

Notwithstanding the above, there is still a lack of low molecular weight fluorescent compounds which can be used as labels for the covalent labelling of target molecules and which will provide multicolour fluorescence detection using single wavelength excitation. There is also a requirement for several such fluorescent labels, each of which can be excited optimally at a particular laser wavelength but fluoresce at significantly different emission wavelengths. We have now found a class of low molecular weight fluorescent labels which will provide multicolour fluorescence detection using single wavelength excitation.

Accordingly, the present invention relates to a multiparameter method for detecting target biological compounds present in multiple samples using a set of different fluorescent labels including at least two labels, each said label being capable of excitation by a single wavelength excitation source and each label emitting fluorescence at a different wavelength, said method comprising:

a) incubating each separate sample with a different label from said set of fluorescent labels to provide fluorescently-labelled samples;
b) mixing each of said fluorescently-labelled samples to form a mixture containing all samples;
c) irradiating the mixture with said single wavelength excitation source; and
d) detecting the fluorescence emissions corresponding to each of the different fluorescently-labelled samples;
characterised in that one or more labels of said set of fluorescent labels is a fluorescent labelling complex comprising:

i) a first fluorochrome having first absorption and emission spectra;
ii) a second fluorochrome having second absorption and emission spectra, the wavelength of the emission maximum of said second fluorochrome being longer than the wavelength of the emission maximum of said first fluorochrome, and a portion of the absorption spectrum of said second fluorochrome overlapping a portion of the emission spectrum of said first fluorochrome;
iii) at least one linker for covalently attaching said first and second fluorochromes for transfer of resonance energy between said first and second fluorochromes;
iv) at least one target bonding group capable of forming a covalent bond with said target biological compound;

wherein said target bonding group is a reactive group for reacting with a functional group of the target compounds; and wherein the combined molecular weight of said first and second fluorochromes and said linker is less than 20,000 Daltons.

Preferably at least one of said first or second fluorochromes is a cyanine dye.

The linker may be rigid or flexible to orientate the transition moments of the donor and acceptor chromophores. For optimal energy transfer to occur, the transition moments of the first and the second fluorochromes are orientated relative to each other in a non-perpendicular direction, eg. positioned generally parallel or in tandem relative to each other. The transition moments of the flexibly linked fluorochromes will change as the linker flexes, but provided that the donor and acceptor transition moments are non-perpendicular during the excited state lifetime of the donor, energy

transfer will occur. The complexes prepared and described herein show energy transfer ranging from 50% to 99% efficiency. Energy transfer efficiency depends on several factors such as spectral overlap, spatial separation between donor and acceptor, relative orientation of donor and acceptor molecules, quantum yield of the donor and excited state lifetime of the donor. In a preferred embodiment, the fluorochromes may be separated by a distance that provides efficient energy transfer, preferably better than 75%.

Closer proximity of the donor and acceptor fluorophores would enhance energy transfer, since efficiency of energy transfer varies as the inverse $6^{th}$ power of separation of the centres of the chromophores according to Forster's equation:

$$ET \propto K^2\ \Phi_D\ J/R^6\ \tau_D$$

where ET is the energy transfer rate constant, $K^2$ is the relative orientation of donor and acceptor transition moments, $\Phi_D$ is the quantum yield of the donor molecule, R is the distance between the centres of the donor and acceptor fluorochromes, J is the overlap between the emission spectrum of the donor and the absorption spectrum of the acceptor fluorochromes, and $\tau_D$ is the excited state lifetime of the donor molecule. See, Forster, T. "Intermolecular Energy Transfer and Fluorescence", Ann. Physik., Vol.2, p.55, (1948). The distance R between the centres of the donor and acceptor fluorochromes may be preferably from 10 to 80 Angstroms. The linker should permit resonance energy transfer between the fluorochromes. The fluorochromes should not interact chemically or form secondary bonds with each other. The linker may be preferably from 2 to 20 bond lengths. For example, if the linker contains an alkyl chain, $-(CH_2)_n-$, the carbon number "n" may be from 1 to about 15. The linker may include part of the constituents extending from the fluorochrome. In other words, the linker is attached to the dye chromophore but is not a part of it. Referring to the linkers shown in Table 2, some extend from the ring nitrogen in one cyanine to a functional group on the benzene ring of another cyanine. Some linkers extend between functional groups on the benzene rings of linked dyes. However, in these examples, none of the linkers includes a network of double bonds that permit conjugation of the donor and acceptor. With a relatively short linker and optimal orientation, there may be efficient resonance energy transfer even when the spectral overlap becomes small. Therefore, it is possible to obtain large wavelength shifts even when only two chromophores are used in the complex.

Suitable linkers are selected from the group consisting of alkyl chains containing from 1 to 20 carbon atoms which may optionally include from 1 to 8 oxygen atoms as polyether linkages, or from 1 to 8 nitrogen atoms as polyamine linkages, or from 1 to 4 CO-NH groups as polyamide linkages, up to 2 bicyclo[2,2,2]octyl groups and up to 10 nucleotide units.

The complexes of the present invention include a target bonding group capable of forming a covalent bond with a target compound to enable the complex to label the target, such as a carrier material or a biological compound. The target bonding group may be a reactive group for reacting with a functional group on the target material. Alternatively the complex may contain a functional group and the target may contain the reactive constituent.

Suitably, the reactive group is selected from the group consisting of succinimidyl ester, isothiocyanates, dichlorotriazine, isocyanates, haloacetamide, maleimide, sulphonyl halides, acid halides, alkylimido esters, arylimido esters, substituted hydrazines, substituted hydroxylamines, carbodiimides and phosphoramidites.

Suitably, the functional group is selected from the group consisting of amino, sulphydryl, carboxyl, hydroxyl, carbonyl, thiophosphate.

Suitably, halo- and halide are selected from chloro, bromo and iodo, or chloride, bromide and iodide.

Suitable target materials may include antibodies, antigens, proteins, carbohydrates, lipids, nucleotides derivatized to contain one of amino, hydroxyl, sulphydryl, carboxyl, or carbonyl groups, and oxy or deoxy polynucleic acids derivatized to contain one of amino, hydroxyl, thiophosphoryl, sulphydryl, carboxyl, or carbonyl groups, cells, polymer particles, or glass beads. In the alternative embodiment, the target may be derivatized to contain the reactive groups identified above to form covalent bonds with the functional groups on the complex.

In a second embodiment, the fluorescent complexes of the invention may contain a polymerizable group suitable for the formation of a polymer containing the complex. Suitable polymerizable groups are selected from acrylate, methacrylate and acrylamide. Polymerization may be carried out with a suitably derivatized complex of this present invention used in conjunction with a second polymerizable monomer starting material, such as styrene or vinyltoluene, to form a copolymer containing the fluorescent complex.

Alternatively, the fluorescent complexes of the invention need not have a reactive group when used to non-covalently bind to another material. For example, the complex may be incorporated during polymerisation or particle formation or may be absorbed into or onto polymer particles.

The complex may also include water solubilising constituents attached thereto for conferring a hydrophilic characteristic to the complex. They are preferably attached to the aromatic ring system of the cyanine fluorochrome. If the cyanine dye does not contain the water solubilising constituent, then the other dye or the linker moiety can contain

the water solubilising group. The water solubilising constituents must be unreactive with the target bonding group of the complex. Suitable solubilising constituents may be selected from the group consisting of amide, sulphonate, sulphate, phosphate, quaternary ammonium, hydroxyl, guanidinium and phosphonate. Sulphonate or sulphonic acid groups attached directly to the aromatic ring of the cyanine fluorochrome are particularly preferred. Water solubility may be necessary when labelling proteins and oxy and deoxy nucleic acids derivatized with amino groups or sulphydryl groups in aqueous solutions. Alternatively, a less hydrophilic polar form of the energy transfer compound may bind non-covalently to DNA by intercalation between the base pairs or by interaction in the minor groove of DNA. Such compounds may be useful for DNA quantitation or localisation.

In addition to the embodiment of the invention which includes a single donor and a single acceptor fluorochrome, the fluorescent labelling complex may include further fluorochromes. The further fluorochromes must have absorption or emission spectra which permit energy transfer to occur. For example, a third fluorochrome may be attached to the second fluorochrome. In this example, the wavelength of the emission spectrum of the third fluorochrome is longer than the wavelength emission of the second fluorochrome, and a portion of the emission spectrum of the second fluorochrome overlaps a portion of the absorption spectrum of the third fluorochrome for transferring energy absorbed from the first fluorochrome to the second fluorochrome to the third fluorochrome.

In another embodiment of the present invention, the complex may include a plurality of the first fluorochromes, each covalently linked by a linker moiety to the second fluorochrome and each capable, upon excitation with light, of transferring energy to the second fluorochrome. In a further embodiment of the present invention, the complex may include a plurality of the second fluorochromes, each covalently linked by a linker moiety to a first fluorochrome and each capable of accepting energy from the first fluorochrome when the first fluorochrome is excited by light. The plurality of first and second fluorochromes may be the same molecule or may be different. For example, there may be several donor fluorochromes which are each excitable at different wavelengths to accommodate different excitation light sources.

In a still further embodiment of the present invention, the complex may include one or a plurality of the second fluorochromes, each covalently linked by a linker moiety to one or a plurality of the first fluorochrome and each covalently linked by a linker moiety to a third fluorochrome. Energy transfer proceeds in parallel in these embodiments.

The first fluorochrome preferably has an extinction coefficient greater than 20,000 Litres/mole.cm and more preferably greater than 50,000 Litres/mole.cm. The second fluorochrome has a fluorescence quantum yield greater than or equal to about 0.05. Quantum yield is generally related to a molecule's rigidity or planarity and indicates the molecule's propensity to fluoresce, ie. give off energy as light, rather than as heat when energy is provided to the molecule.

The complexes of the present invention preferably include at least one cyanine fluorochrome and preferably at least one polymethine cyanine dye. The cyanines are particularly useful due to the wide range of structural variations and spectral properties available that may be obtained by varying the number of carbon atoms in the methine bridge, and the heteroatoms or other constituents of the cyanine dyes. It is possible to synthesise dyes having particular excitation wavelengths to correspond to a particular excitation source, such as a laser, eg. a HeNe laser or a diode laser. Therefore, energy transfer labels can be made that absorb and emit efficiently at most wavelengths in the visible region of the spectrum. Commonly used sources of excitation excite at laser line 488nm. Whilst that excitation wavelength will be used for the purposes of the description of the invention, it is to be understood by those skilled in the art that other energy transfer labels can be made for specific excitation sources without departing from the scope of the invention.

Examples of dyes that can be used as donor and acceptor fluorochromes in the fluorescent labelling complexes of the present invention are shown in formulas 2 and 3,

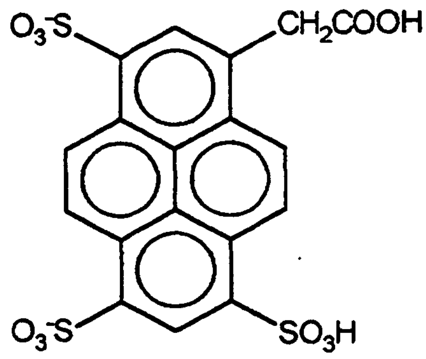

Cascade Blue (2)

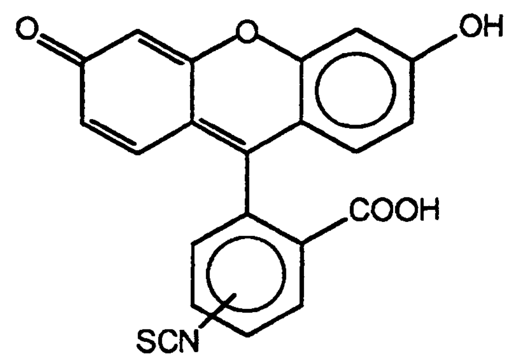

FITC (3)

and in formula (4),

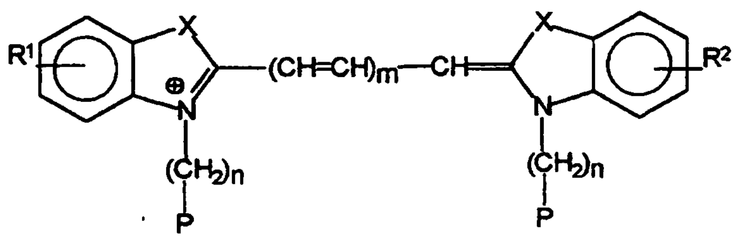

(4)

wherein X is selected from $C(CH_3)_2$, sulphur and oxygen, $R^1$ and $R^2$ are independently selected from the group consisting of $CH_2NH_2$, $SO_3^-$, $CH_2COOH$ and NCS, P is selected from $SO_3^-$, $NH_2$ and COOH, m = 1,2, or 3 and n is an integer from 1-5.

Additional cyanines for use in complexes of the invention are the rigidized monomethine cyanines disclosed in the copending application of Waggoner et al, entitled "Rigidized Monomethine Cyanines", filed on even date herewith. The monomethine rigidized dyes have the following general structure (5).

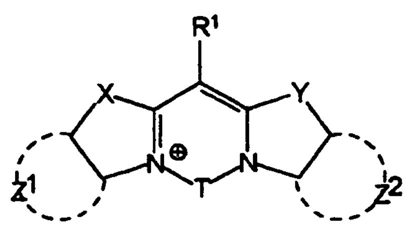

(5)

optionally substituted by one to six groups $R^2$ to $R^7$;

where T is a linking group such that:

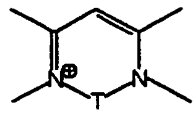

is a six or seven membered ring;

X and Y are selected from bis-substituted carbon, oxygen, sulphur, selenium, -CH=CH-, and - N-W wherein N is nitrogen and W is selected from hydrogen and a group $-(CH_2)_nR^8$ where n is an integer from 1 to 26 and $R^8$ is selected from hydrogen, amino, aldehyde, acetal, ketal, halo, cyano, aryl, heteroaryl, hydroxyl, sulphonate, sulphate, carboxylate, substituted amino, quaternary amino, nitro, primary amide, substituted amide, and groups reactive with amino, hydroxyl, aldehyde, phosphoryl, or sulphydryl groups;

groups $Z^1$ and $Z^2$ represent the atoms necessary to complete one, two fused or three fused aromatic rings each ring having five or six atoms, selected from carbon atoms and, optionally, no more than two oxygen, nitrogen and sulphur atoms; and

$R^2$ and $R^3$ are attached to the carbon atoms of T when T contains carbon atoms.

The rigidized monomethine cyanine dyes have sharp distinct absorptive and emissive signals, which are photostable. Certain of the rigidized monomethine cyanine dyes maximally absorb and emit light at wavelengths between 300 and 500nm.

Other low molecular weight fluorochromes in addition to the cyanine fluorochromes may be selected from the fluoresceins, pyrene trisulphonates (which are sold under the trade mark "Cascade Blue"), rhodamines, and derivatives of the bis-pyrromethine boron difluoride dyes, such as 3,3',5,5'-tetramethyl-2,2'-pyrromethene-1,1'-boron difluoride, sold under the trademark BODIPY by Molecular Probes Inc. BODIPY analogues are disclosed in US Patent Nos. 4774339, 5187223, 5248782 and 5274113 (Haugland and Kang), as well as in the "Handbook of Fluorescent Probes and Research Chemicals", published by Molecular Probes Inc.

For obtaining exceptionally large excitation-emission wavelength shifts, it is possible to use sequential energy transfer steps in the complex. For example, three chromophores have been linked to provide maximal emission at the wavelength of a cyanine dye, the heptamethine cyanine, CY7, (compound 4, $X=C(CH_3)_2$, $R^1$, $R^2=-SO_3^-$, P=COOH, n=5, m=3), above 780nm with excitation at 488nm. The initial donor was fluorescein isothiocyanate and the intermediate fluorophore in the complex was the trimethine cyanine dye designated CY3 (compound 4, $X=C(CH_3)_2$, $R^1=R^2=CH_2NH_2$, $P=SO_3^-$, n=4, m=1). The fluorescein was excited at 488nm and transferred nearly 100% of its excited state energy to the trimethine cyanine, which in turn transferred about 90% of its excited state energy to the CY7 fluorescing at 782nm. The same efficiency was observed when a pentamethine cyanine CY5 was used in place of CY7, with fluorescence at 667nm. The development of such multichromophore complexes is particularly useful for multicolour detection systems.

Although several of the complexes show efficient energy transfer, the overall quantum yield of these labelling complexes can be further improved. For example, the use of acceptor dyes with quantum yield higher than that of CY5 would improve the overall brightness of the complex.

The fluorescent labelling complexes of the invention have low molecular weights and can be readily conjugated to antibodies, other proteins and DNA probes. Low molecular weight as used herein shall mean that the combined molecular weight of the fluorochromes and linker of the complex is preferably between about 500 and 10000 Daltons, and for the two fluorochrome complex, preferably in the range of 1000 to 2500 Daltons. Therefore these labelled species will have much greater penetration into intracellular environments than is possible with the large phycobiliprotein labels currently in use. The low molecular weight fluorescent complexes of the present invention should be valuable not only for flow cytometry, but also for laser confocal microscopy and for other detection systems requiring multicolour detection with single wavelength excitation.

The invention includes a reagent and a method for making the reagent including incubating the fluorescent water soluble labelling complex described above with a carrier material.

The present invention also provides processes for the preparation of the fluorescent labelling complexes which comprise covalently linking fluorochromes such as cyanine fluorochromes to cyanines or other fluorochromes, by methods well known to those skilled in the art to form energy transfer donor-acceptor complexes.

For example, complexes of the present invention wherein the linkage contains an amide or an ester may be prepared by the reaction of a compound of formula (6) with a compound of formula (7);

R-(M)-COA B-(N)-R'

(6) (7)

wherein R and R' are different fluorochromes; COA is an activated or activatable carboxyl group; B is $NH_2$ or OH; and M and N are independently aliphatic moieties containing $C_{1-12}$ alkyl and optionally including one or more linking phenyl, naphthyl, amide, ester, or ether functionalities. See for example, Mujumdar, R.B. et al, Bioconjugate Chemistry, Vol.4, pp.105-111, (1993); US Patent No.5268486 to Waggoner et al. Suitable groups A include halo, for example chloro or bromo, para-nitrophenoxyl, N-hydroxysuccinimido, or OCOR" wherein R" is $C_{1-6}$ alkyl.

Complexes of the present invention wherein the linkage contains an amino, ether or a thioether group, may be prepared by the reaction of a compound of formula (8) with a compound of formula (9);

R-(M)-B' C-(N)-R'

(8) (9)

wherein R, R', M and N are as defined above; B' is OH, $NH_2$, or SH; and C is a displacable group for example iodo, or para-toluenesulphonate. The reaction is suitably carried out in the presence of a base.

Alternatively, complexes of the present invention may be prepared by first coupling together two dye precursors using a non-conjugated linker to give an intermediate represented by structure (10).

Xa-(L)-Xb

(10)

wherein Xa and Xb are independently substituted or unsubstituted heterocyclic precursors and (L) is a non-conjugated linker group comprising $C_{1-12}$ alkyl, optionally including one or more linking phenyl, naphthyl, bicyclo[2,2,2]octyl, ether, amine, ester, or amide groups, or combinations thereof. Suitable heterocyclic precursors, Xa and Xb are shown in Table 1, Compounds I and II. By way of example, the synthesis of intermediate (10) wherein the linker consists of an alkyl chain linked to the nitrogen atoms of two indolenine units, may be accomplished by reaction with an $\alpha,\omega$-dihaloalkane, such as 1,6-dibromohexane, either in a one or a two stage reaction process. Suitably the reaction is carried out at an elevated temperature such as about 100-110°C, in an inert solvent such as xylene. See for example, Hamer, F.M., "The Cyanine Dyes and Related Compounds", p.676, Wiley Interscience (1964).

The intermediate (10) can then be used as a precursor in the formation, by methods known in the art, of complexes containing two different fluorophores connected by the linker. See for example, Hamer, F.M., "The Cyanine Dyes and Related Compounds", p.118-119, Wiley Interscience (1964).

The following examples serve to illustrate the preparation of complexes of the present invention and their spectral properties.

Example 1. **Preparation of CY5-CY7 Complex**

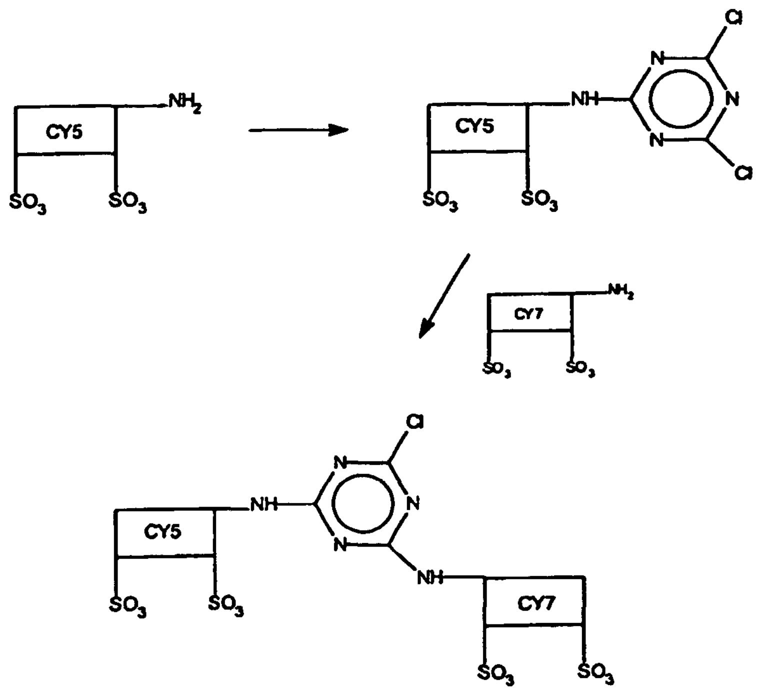

Cyanuric chloride (trichlorotriazine) (5mg), sodium bicarbonate (2mg), and purified dimethylformamide (DMF) (0.25ml) were mixed at 0°C. To this solution was added 5mg of amino-cyanine dye (Mujumdar et al, Cytometry, Vol. 10, pp.11-19, (1989)), represented above by the box containing CY5 and the mixture was stirred at 0°C for 10 minutes. Stirring was continued overnight at room temperature. Thin layer chromatography (TLC) revealed one major spot and two minor spots; the latter spots were determined to be impurities.

The reaction mixture was worked up by precipitation with ether. A dark blue powder was obtained. DMF (0.3ml) was added to dissolve the powder. To this solution was added sodium bicarbonate (2mg) and 4.7mg of the amino-CY7 dye represented by the box containing CY7. The mixture was stirred at room temperature for 24 hours. The product was precipitated and washed several times with ether, providing a dark powder. The complex showed an absorption spectrum with peaks for the individual fluorochromes at 650nm (CY5) and 761nm (CY7), indicating that no new chromophore had been generated.

Example 2. **Synthesis of Complex 1 and Related Compounds (see Table 2)**

i) General Methods

a) Purification of Dyes

Purification of the fluorochromes was performed on a Spectra-Physics model SP8700 analytical HPLC unit equipped with a C8-RP column. Purification could also be achieved by conventional or flash column chromatography on commercially available C18-RP powder. Water/methanol mixtures were used for elution in all experiments. Dyes were recovered from the fractions by rotary evaporation at 60-70°C without appreciable loss. For further purification, the fluorochrome, with undetermined counter ion composition was passed through a Dowex-50W column (hydrogen form).

b) Spectroscopic Measurements and Analytical Determinations

Ultra-violet/visible spectra were measured with a Hewlett-Packard HP8452 diode array spectrophotometer.

Proton NMR spectra were obtained with an IBM 300 FT-NMR spectrometer using $D_2O$, $CD_3OD$ or DMSO-d6 as solvents. NMR signals are described in δ by the use of s for singlet, d for doublet, t for triplet, q for quartet and m for multiplet. Fluorescence measurements were performed using a SPEX Fluorolog 2 System. Quantum yields were determined by known techniques as described by Mujumdar R.B.,et al, "Cyanine Dye Labelling Reagents Containing Isothiocyanate Groups", Cytometry, Vol.10, pp.11-19 (1989).

c) Cell Preparation and Flow Cytometry

Mononuclear leukocytes were obtained by Histopaque, density 1.077, separation from healthy volunteers. The lymphocyte population was selected by flow cytometry based on forward and side scatter characteristics. Subpopulations were identified using specific monoclonal antibodies (CD4, staining T-helper cells and CD3, pan T-cell population). Optimal concentration of Complex 1-tagged antibody was determined by analysing the results of a dilution series. Direct immunofluorescence was accomplished by incubating the recommended amount of labelled antibody with 1-2 x $10^6$ cells for 45 minutes at 4°C. Samples were then washed twice in Hank's balanced salt solution (HBSS) containing 2% fetal bovine serum and 0.1% sodium azide. After the final wash, the cells were resuspended in 1ml of HBSS containing 1% paraformaldehyde and analysed within one week. Flow cytometry measurements were made with a Becton Dickinson FACS 440 dual laser flow cytometer equipped with a Consort 40 data analysis system. The argon ion laser provided 400mW of excitation at 488nm. Fluorescence signals from Complex 1 and R-phycoerythrin were collected using 670/13.5nm and 575/26nm band pass filters respectively.

d) Calculation of Donor Quenching Efficiency (DQE)

Resonance energy transfer efficiencies were estimated from the quenching of donor fluorescence intensities. Absorption and fluorescence spectra of the donor (alone) and the fluorescent labelling complex were obtained in order to determine the relative concentrations of each in fluorescence experiments. Donor excitation was used to obtain emission spectra of both compounds. DQE was then calculated using:

$$DQE\% = (1 - F^{ET}A/FA^{ET}) \times 100$$

where F is the fluorescence intensity of the donor alone, $F^{ET}$ is the fluorescence intensity of the complex at the donor wavelength, A is the absorbance at the wavelength of excitation of the donor alone and $A^{ET}$ is the absorbance at the wavelength of excitation of the fluorescent labelling complex.

e) Synthesis of Fluorochromes

Amino cyanines ($CY3NH_2$, $CY3(NH_2)_2$ and $CY3NH_2SO_3$) and carboxyalkyl cyanines (CY5COOH, $CY3O(SO_3)_2$, $CY5(SO_3)_2$ and $CY7(SO_3)_2$) required as precursors for energy transfer fluorochromes were synthesised by the methods previously described in Ernst, L.A. et al, "Cyanine Dye Labelling Reagents for Sulphydryl Groups", Cytometry, Vol.10, pp.3-10, (1989), Hammer, F.M., "The Cyanine Dyes and Related Compounds", (Wiley, pub. New York 1964), Mujumdar, R.B.et al, "Cyanine Dye Reagents Containing Isothiocyanate Groups", Cytometry, Vol.10, pp.11-19, (1989); Mujumdar, R.B.et al, "Cyanine Dye Labelling Reagents: Sulphoindocyanine succinimidyl ester", Bioconjugate Chemistry, Vol.4, pp.105-111, (1993); Southwick, P.L. et al, "Cyanine Dye Labelling Reagents: Carboxymethylindocyanine succinimidyl esters", Cytometry, Vol.11, pp.418-430, (1990). The synthesis and properties of one amino-cyanine fluorochrome, $CY3NH_2SO_3$ and its conjugation with the succinimidyl ester of $CY5(SO_3)_2$ to form Complex 1 is described below. The spectral properties for all the fluorochromes are shown in Tables 3 and 4. The unsymmetrical trimethinecarbocyanine, $CY3NH_2SO_3$, was synthesised in four steps. Refer to Table 1 for the structures (I) - (VI).

**Table 1**

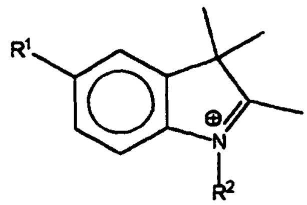

| Compound | $R^1$ | $R^2$ |
|---|---|---|
| I | H | H |
| II | $CH_2$Phth | H |
| III | $CH_2$Phth | $(CH_2)_5COOH$ |
| IV | $SO_3^-$ | $(CH_2)_5COOH$ |

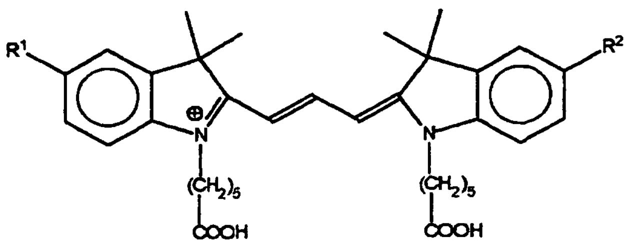

| Compound | $R^1$ | $R^2$ |
|---|---|---|
| V | $SO_3^-$ | $CH_2$Phth |
| VI | $SO_3^-$ | $CH_2NH_2$ ($CY3NH_2SO_3$) |

$CH_2$Phth =

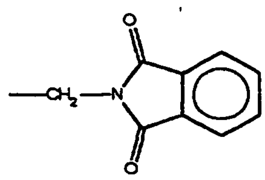

1.5.1 Synthesis of 5-Phthalimidomethyl-1-(ε-carboxypentyl)-2,3,3-trimethylindole (III)

5-Phthalimidomethyl-2,3,3-trimethylindolenine (II) was synthesised according to the procedure of Gale and Wilshire, "The Amidomethylation and Bromination of Fischer's Base. The Preparation of Some New Polymethine Dyes", Aust.J.Chem., Vol.30, pp.689-694, (1977). Powdered N-hydroxymethylphthalimide (70g, 0.4mol) was added in small portions over a period of 45 minutes to a stirred solution of 2,3,3-trimethyl-(3H)-indolenine (I) (70g, 0.44mol) in concentrated sulphuric acid (360ml) at room temperature. The solution was stirred for 70 hrs at room temperature before being poured onto ice-water. Basification of the solution with conc. ammonium hydroxide gave a yellow powder which

was filtered and dried (111g, yield 80%, mp.180-182°C). $^1H$ NMR (DMSO-$d_6$), δ, 7.8-7.95 (m, 4H, phthalimido), 7.4 (s, 1H, 4-H), 7.38 (d, 1H, J=9.0Hz, 6-H), 7.2 (d, 1H, J=9.0Hz, 7-H), 4.7 (s, 2H, -$CH_2$), 2.2 (s, 3H, $CH_3$), 1.2 (s, 6H, -$(CH_3)_2$).

This dry powder (10g, 0.03mol) and 6-bromohexanoic acid (9.1g, 0.05mol) were mixed in 1,2-dichlorobenzene (25ml) and heated at 125°C for 12 hours under nitrogen. The mixture was cooled. 1,2-Dichlorobenzene was decanted and the solid mass was triturated with isopropanol until free powder was obtained (11g, yield 80%, mp. 124-126°C). $^1H$ NMR (DMSO-$d_6$), δ, 7.8-7.95 (m, 4H, phthalimido), 7.4 (s, 1H, 4-H), 7.38 (d, 1H, J=9.0Hz, 6-H), 7.2 (d, 1H, J=9.0Hz, 7-H), 4.7 (s, 2H, -$CH_2$), 4.5 (t, 2H, J=7.5Hz, α-$CH_2$), 2.3 (t, 2H, J=7Hz, ε-$CH_2$), 1.99 (m, 2H, β-$CH_2$), 2.3-1.7 (m, 4H, γ-$CH_2$ and δ-$CH_2$ merged with s of 6H-$(CH_3)_2$).

1.5.2 <u>Synthesis of 1-(ε-carboxypentyl)-2,3,3-trimethylindoleninium-5-sulphonate (IV)</u>

Compound (IV) was synthesised according to the procedure described previously by Mujumdar, R.B. et al, Bioconjugate Chemistry, (1993), *supra*. The potassium salt of 2,3,3-trimethylindoleninium-5-sulphonate (11g, 0.04mol) and 6-bromohexanoic acid (9.8g, 0.05mol) were mixed in 1,2-dichlorobenzene, (100ml) and heated at 110°C for 12 hours under nitrogen. The mixture was cooled. 1,2-Dichlorobenzene was decanted and the solid mass was triturated with isopropanol until free powder was obtained (11g, yield 80%). λmax (water) 275nm: $^1H$- NMR ($D_2O$), δ, 8.13 (s, 1H, 4-H), 8.03 (dd, 1H, J=9.0, 1.1Hz, 6-H), 7.2 (d, 1H, J=9.0Hz, 7-H), 4.51 (t, 2H, J=7.5Hz, α-$CH_2$), 2.25 (t, 2H, J=7.5Hz, γ-$CH_2$), 1.99 (m, 2H, β-$CH_2$), 1.35-1.66 (m, 4H, δ-$CH_2$, γ-$CH_2$), 1.61 (s, 6H, -$(CH_3)_2$). $R_f$ = 0.55 (C-18, water-methanol, 25%).

1.5.3 <u>Synthesis of Intermediate (V)</u>

A solution of 1-(ε-carboxypentyl)-2,3,3-trimethylindoleninium-5-sulphonate (IV) (10g, 0.03mol) and N,N-dimethylformamide (7.2g, 0.04mol) in acetic acid (20ml) were heated to reflux for 1 hour. Acetic acid was removed on a rotary evaporator and the product was washed with ethyl acetate (3x50ml) whereupon a dark brown solid was obtained. λmax (water) 415nm $R_f$ = 0.32 (C-18, 25% methanol in water). The crude product thus obtained was used for the next reaction without further purification. The solid (3.8g) was dissolved in a mixture of acetic anhydride (10ml) and pyridine (5ml). 5-Phthalimidomethyl-1-(ε-carboxypentyl)-2,3,3-trimethylindole (III) (2.5g, 6mmol) was added and the reaction mixture was heated to 110°C for 1 hour. The solution was cooled and diluted with diethyl ether (500ml). Product separated in the form of a red powder from which supernatant fluid was removed by decanting. It was dissolved in a minimum volume of methanol and re-precipitated with 2-propanol. The product was collected on a filter paper and dried to yield 5.3g of compound (V). It was purified by flash column chromatography on reverse phase C-18 using water methanol mixture as eluent, (1.6g, yield 30%). λmax (water) 554nm; e $1.3x10^5$ L/mol.cm. $^1H$ NMR ($CD_3OD$), δ, 8.5 (t, 1H, J = 14 Hz, β-proton of the bridge), 7.8-8.0 (m, 6H, 4 protons of the phthalimido group and 4-H and 6-H of the sulphoindole ring), 7.55 (s, 2H, 4'-H), 7.6 (d, 1H, J=12Hz, 6'-H), 7.3 (two d, 2H, 7-H and 7'-H), 6.1-6.3 (t, 2H, α, α'-protons of the bridge), 4.1 (m, 4H, α, α'-$CH_2$-), 2.9 (t, 2H, J = 7Hz, -$CH_2COOH$), 1.4-2.0 (m, 21H, three -$CH_2$, one -$CH_3$, and two -$(CH_3)_2$, methyl protons of the phthalimidomethyl group are merged in a water signal at 4.8.

1.5.4 <u>Hydrolysis of (V) to give (VI)</u>

Compound (V) (1g, 1.1mmol) was dissolved in concentrated hydrochloric acid (5ml) and heated under reflux for 12 hours. After cooling, the crystalline phthalic acid was filtered off The filtrate was concentrated with a rotary evaporator and then slowly neutralised with concentrated ammonium hydroxide while the temperature was kept below 30°C. Pure fluorochrome $CY3NH_2SO_3$ (VI) was obtained by reverse phase column chromatography using a water-methanol mixture as eluent. λmax (methanol) 552nm. $^1H$ NMR (DMSO-$d_6$), δ, 8.45 (t, J = 7.2Hz, 1H, 9-H), 7.3-7.9 (m, 6H, aromatic protons), 6.55 (dd, 2H, 8 and 8'-H), 4.5 (m, 4H, N-$CH_2$), 4.1 (s, 2H, $CH_2NH_2$), 2.15 (t, 2H, $CH_2COOH$), α, α'-protons of the bridge), 4.1 (m, 4H, α, α'-$CH_2$-), 2.9 (t, 2H, J = 7Hz, -$CH_2COOH$), 1.25-1.8 (broad m, 24H, two -$(CH_2)_2$ and 6-C-$(CH_3)_2$). $R_f$ = 0.415 (RP C18 60% methanol in water).

1.5.5 <u>Synthesis of Complex I</u>

Dry powder of $CY5(SO_3)_2$ succinimidyl ester (425mg, 0.26mmol) prepared by the method of Mujumdar et al, Bioconjugate Chemistry, Vol.4, pp.105-111, (1993), was added in small portions to a well stirred solution of $CY3NH_2SO_3$ (200mg, 0.26mmol) in 10ml of carbonate - bicarbonate buffer (0.1M, pH 9.4). Stirring was continued for an additional 30 minutes after which the reaction was purified by flash column chromatography on C-18 reverse phase powder using water-methanol (6.3:3.7) as eluent. 5ml fractions were collected and monitored by TLC. Fractions containing CY5 $(SO_3)_2$ acid and $CY3NH_2SO_3$ were discarded. Violet coloured fractions were checked by ultraviolet light in methanol and the fractions containing Complex 1 fluorochrome (Table 2) were pooled. Evaporation of the solvent yielded Complex

1 as a violet powder, (yield 37%). Rf = 0.45 (RP 37% methanol-water). $^1$H NMR spectrum recorded in $D_2O$ showed broad signals and were difficult to assign. The fluorochrome was purified on a strongly acidic ion-exchange column (Dowex 50, $H^+$ form). High resolution FAB mass spectrometry showed $(M+H)^+$ ion at 1391.83 ($C_{73}H_{91}N_5O_{16}S_3$ +H requires 1391.73).

1.5.6 Succinimidyl Ester of Energy Transfer Cyanine Dye

Complex 1 (60mg, 0.04mmol) was dissolved in a mixture of dry DMF (1ml) and dry pyridine (0.05ml). Disuccinimidyl carbonate (DSC) (46mg, 0.18mmol, 1.5 equiv/carboxyl group) was added and the mixture was stirred at 55-60°C for 90 minutes under nitrogen. After diluting the mixture with dry diethyl ether (20ml), the supernatant was decanted. The product was washed repeatedly with ether, filtered and dried under vacuum. The formation of the active succinimidyl ester was confirmed by its reaction with benzylamine in DMF or its reaction with taurine in a pH 9.4 bicarbonate buffer. Reversed phase C-18 TLC spotted with the conjugate, the succinimidyl ester and the hydrolysed carboxylate product for comparison was developed with water-methanol (1:1) mixture. $R_f$ = 0.78 (Acid), 0.3 (Benzylamine adduct).

1.5.7 Reaction of Succinimidyl Ester with Antibody and Streptavidin

A stock solution of Complex 1 fluorochrome succinimidyl active ester was made in dry DMF (1mg/100μl). In one sample, one milligram sheep (γ-globulin was dissolved in 0.25ml carbonate/bicarbonate buffer (approximately 6.45nmol/0.25ml). In another example, streptavidin (1mg) was dissolved in 0.25ml of the carbonate/bicarbonate buffer. Appropriate volumes of the fluorochrome stock were added to 0.25ml portions of each protein solution to produce the desired starting fluorochrome to antibody ratios, and each reaction mixture was stirred at room temperature for 30 minutes. The protein conjugate was separated from unreacted fluorochrome in each sample by gel filtration chromatography over Sephadex G-50 (0.7x20cm column), using PBS, pH 7.4, containing 0.1% azide. Dye conjugated proteins eluted as coloured bands well separated from the unreacted fluorochrome. The normalised excitation spectrum of the Complex 1-streptavidin conjugate in PBS is shown in Figure 4. The absorbance spectrum of Complex 1-Sheep IgI in PBS is shown in Figure 5. Figure 6 shows the flow cytometry analysis of Complex 1-streptavidin used to detect CD3 antibody.

Further energy transfer donor acceptor complexes according to the present invention were prepared from cyanine fluorochromes in order to investigate the energy transfer efficiency of such compounds. The structures of these analogues are shown in Table 2.

The spectral properties of the precursor cyanines are given in Table 3 and those of the complexes are shown in Table 4.

**Table 2**

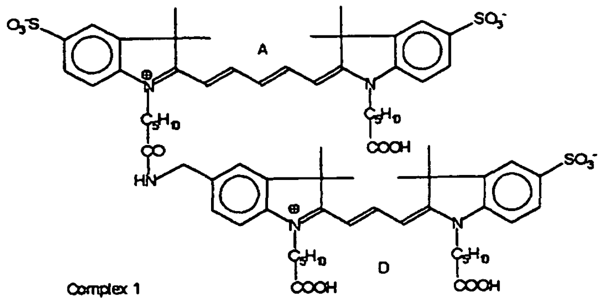

Complex 1

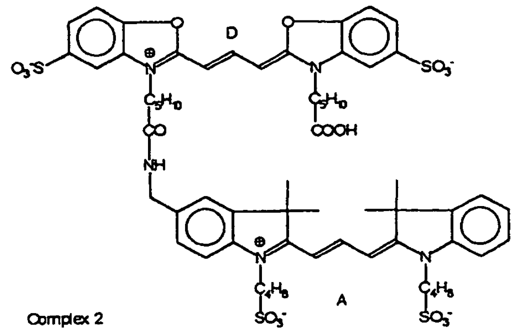

Complex 2

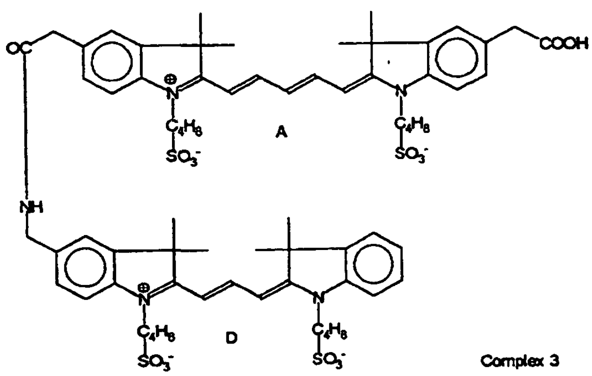

Complex 3

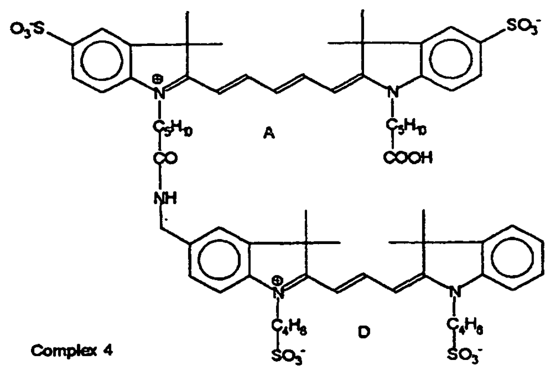

Complex 4

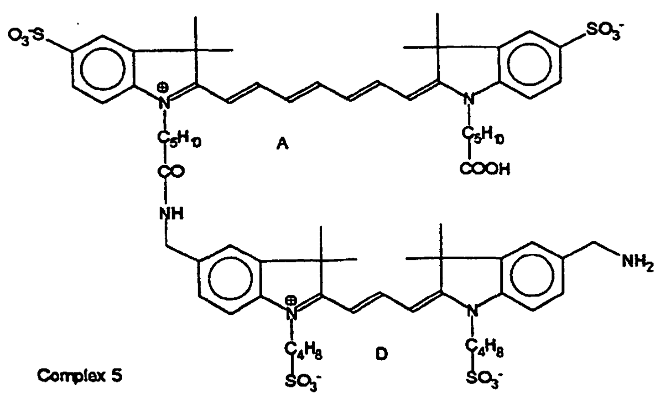

Complex 5

**Table 2 (continued)**

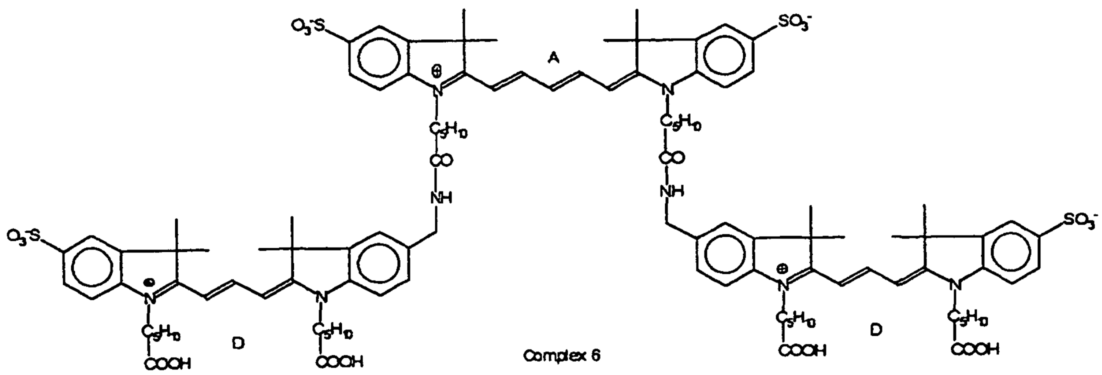

Complex 6

**"A" designates the fluorochrome that acts as the energy acceptor and "D" designates the fluorochrome that acts as the energy donor.**

The energy transfer complexes shown in Table 2 are as follows: Complex 1, $CY3NH_2SO_3$ (Donor) + CY5 $(SO_3)_2$ (Acceptor) ; Complex 2, CY3-$O(SO_3)_2$ (Donor) + $CY3NH_2$ (Acceptor); Complex 3, $CY3NH_2$ (Donor) + CY5COOH (Acceptor) ; Complex 4, $CY3NH_2$ (Donor) + $CY5(SO_3)_2$ (Acceptor); Complex 5, $CY3(NH_2)_2$ (Donor) + CY7 $(SO_3)_2$ (Acceptor) ; Complex 6, 3 $CY3NH_2SO_3$ (Donor) + $CY5(SO_3)_2$ (Acceptor).

Table 3

| **Spectral Properties of Cyanine Dyes Used as Precursors for the Fluorescent Energy Transfer Complexes of the Invention** | | | | |
|---|---|---|---|---|
| Dye | Solvent | Absorption Maximum (nm) | Emission Maximum (nm) | Quantum Yield (M) |
| Amine containing Cyanine Dyes | | | | |
| $CY3NH_2$ | Methanol | 552 | 569 | 0.05 |
| | PBS | 548 | 563 | 0.05 |
| $CY3(NH_2)_2$ | Methanol | 552 | 569 | 0.05 |
| | PBS | 548 | 653 | 0.05 |
| $CY3NH_2SO_3$ | Methanol | 556 | 573 | 0.08 |
| | PBS | 548 | 653 | 0.09 |
| Carboxyl containing Cyanine Dyes | | | | |
| CY5COOH | Methanol | 658 | 685 | 0.22 |
| | PBS | 648 | 667 | 0.13 |

Table 3 (continued)

| Spectral Properties of Cyanine Dyes Used as Precursors for the Fluorescent Energy Transfer Complexes of the Invention | | | | |
|---|---|---|---|---|
| Dye | Solvent | Absorption Maximum (nm) | Emission Maximum (nm) | Quantum Yield (M) |
| Carboxyl containing Cyanine Dyes | | | | |
| $CY5(SO_3)_2$ | Methanol | 658 | 677 | 0.4 |
| | PBS | 650 | 667 | 0.27 |
| $CY3\text{-}O(SO_3)_2$ | Methanol | 492 | 506 | 0.2 |
| | PBS | 486 | 500 | 0.09 |
| $CY7(SO_3)_2$ | Methanol | 758 | 789 | ND[a] |
| | PBS | 750 | 777 | ND[a] |

[a]ND means not determined. PBS means phosphate-buffered saline.

The efficiency of energy transfer was estimated by calculating the amount of quenching of donor fluorescence that occurs (DQE) when the acceptor is attached. It is possible that some quenching could occur by pathways other than resonance energy transfer when the acceptor is bound. However, the cyanine donor preferred for the fluorescent labelling complexes of the present invention are relatively insensitive to their molecular environment. Furthermore, addition of large substituents to trimethine cyanines usually increases, rather than decreases, their fluorescence. Therefore, DQE may be equal to the efficiency of energy transfer. The estimated energy transfer efficiencies based on DQE measurements ranged 50% to 99% and the wavelength shifts between the donor absorption maxima and the terminal acceptor emission maxima (DI) varied between 83nm and 294nm.

Two of the complexes, 1 and 6, are capable of absorbing light at the argon laser wavelength, 488nm. Complex 1 contains a single donor and single acceptor, and Complex 6 contains 2 donors per acceptor. Complex 1 has 3 carboxyl groups and Complex 6 has 4 carboxyl groups. These are converted to succinimidyl active esters upon activation. Figure 2 shows the absorption spectra of Complex 1 and Complex 6 in methanol.

Complex 1 was selected for further studies. As shown in Figures 3(a) and 3(b), the absorbance (solid line) of Complex 1 varies slightly in phosphate-buffered saline (Figure 3(b)) and methanol (Figure 3(a)) but fluorescence remains unchanged. The emission of the donor component at 572nm is very weak compared with the emission of the acceptor at 675nm, as would be expected when energy transfer is efficient.

Figure 5 demonstrates that sheep antibodies can be readily labelled with the activated Complex 1. Conjugates made of Complex 1 conjugated to sheep IgG at various dye:protein ratios were tested. The lowest dye:protein ratio is represented by the line having its first peak (at about 270nm) at 0.8 and the highest dye:protein ratio is represented by the line having its first peak (at about 270nm) at a little less than 0.4. No dimer formation involving either the donor or the acceptor fluorochromes was observed with increasing dye:protein ratios. Each Complex 1 contains up to 3 reactive groups. More reactive groups may be used provided no cross-linking occurs. It is important to use labelling conditions that avoid protein cross-linking which quench the fluorescence. Cross-linking by doubly activated cyanines has been observed previously by Southwick, P.L. et al, "Cyanine Dye Labelling Reagents: Carboxymethylindocyanine succinimidyl esters", Cytometry, Vol.11, pp 418-430, (1990) and can be minimised by limiting the concentration of protein to be labelled to approximately 1mg/ml.

Upon binding to antibodies, the quantum yield of the complex was enhanced three fold as shown in Table 4. It is believed that this occurs because the radiationless deactivation pathway of both the CY3 and CY5 components of Complex 1 are reduced because of their restricted mobility when bound to the surface of the protein. Other means of restricting conformational mobility are known to increase the fluorescence efficiency of cyanine fluorochromes, as described in Mujumdar, R.B. et al, "Cyanine Dye Labelling Reagents: Sulphoindocyanine Succinimidyl Ester", Bioconjugate Chemistry, Vol.4, pp.105-111, (1993).

In fact, when Complex 1 was dissolved in glycerine, the quantum yield increased by several fold, as shown in Table 4.

Activated Complex 1 can be used as a fluorescent label for 2 colour flow cytometry experiments with 488nm excitation. The scatter plot is shown in Figure 6. Human T-lymphocytes were used to compare the Complex 1 label with another two-colour reagent, R-phycoerythrin, which also excites at 488nm and emits at 575nm. Complex 1 labelled streptavidin (fluorochrome/protein ~4) was used to detect biotinylated CD3 antibody, which marks all T-cells. In the same lymphocyte sample, phycoerythrin(PE)-labelled anti-CD4 was used to mark the Helper Cell subset of the T-cells.

Thus, in the total lymphocyte population there is a population of cells that contain neither CD3 nor CD4 (ie. CD3 and CD4 negative, in the lower left population of the 2-dimensional scatter plot in Figure 6), a subset of Complex 1-labelled CD3-positive cells that do not have a phycoerythrin signal (ie. CD3 positive and CD4 negative, shown in the upper left population of Figure 6), and a third subset consisting of Complex 1-labelled cells that are phycoerythrin stained (ie. CD3 and CD4 positive, shown in the upper right population of Figure 6). It is clear that Complex 1 gave base-line separation of the positive and negative cell populations, and that there was minimal spill over of Complex 1 fluorescence into the phycoerythrin channel. The Complex 1 fluorochrome gave a three times brighter signal when the fluorochrome was excited at 514nm.

Example 3

Several other complexes with the general structure shown in formula (10) below were synthesised. Table 5 shows their spectral properties in solution in methanol.

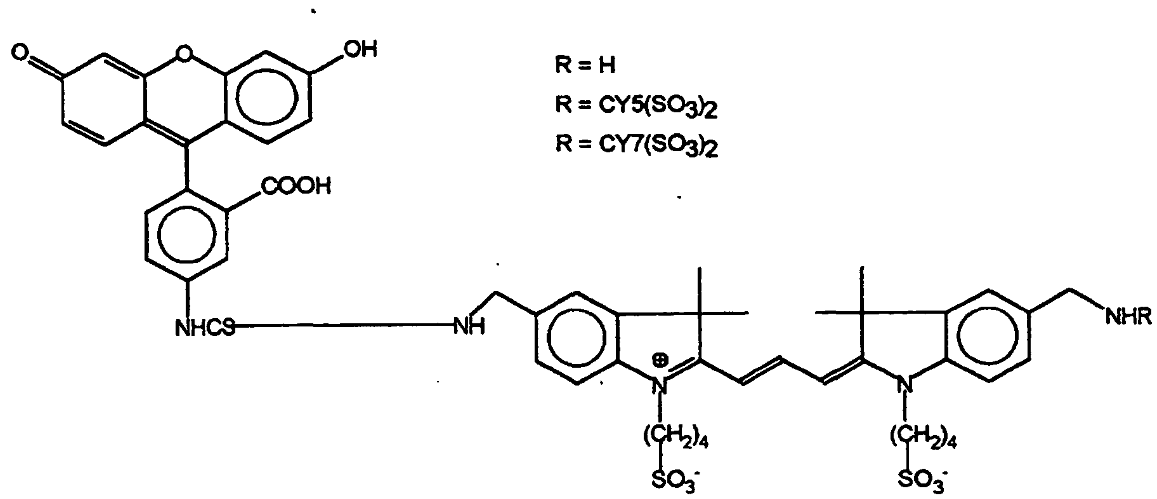

R = H

R = $CY5(SO_3)_2$

R = $CY7(SO_3)_2$

(10)

These series of spectra demonstrate efficient energy transfer with long Stokes' shifts. Each emission spectrum shows substantially all of the emission coming from the final acceptor fluorochrome in each series with only minimal emission from either the donor fluorescein, or the intermediate cyanine.

Table 4

| **Spectral Properties of Energy Transfer Complexes** | | | | | | |
|---|---|---|---|---|---|---|
| Dye | Abs max (nm) | Excitation Wavelength (nm) | Em max (nm) | Quantum Yield (M) | Energy Transferred (%) | Wavelength Shift [e] (nm) |
| Complex 1[a] | 556 (9.5), | 488 | 675 | 0.32 | 91 | 119 |
| | 652 (14.3) | 514 | 676 | 0.37 | 92 | 120 |
| | | 600 | 673 | 0.49 | - | - |
| Complex 1[b] | 536 (16), | 488 | 675 | 0.03 | 89 | 139 |
| | 658 (16) | 514 | 673 | 0.04 | 89 | 137 |
| | | 600 | 668 | 0.21 | - | - |

[a] = in methanol,

[b] = in PBS,

[e] = difference between $Em_{max}(A) - Ab_{max}(D)$

Table 4 (continued)

| Spectral Properties of Energy Transfer Complexes | | | | | | |
|---|---|---|---|---|---|---|
| Dye | Abs max (nm) | Excitation Wavelength (nm) | Em max (nm) | Quantum Yield (M) | Energy Transferred (%) | Wavelength Shift [e] (nm) |
| Complex 1[c] (PBS) | 558, 658 | 488 | 674 | 0.11 | 95 | 116 |
| | | 514 | 673 | 0.13 | 95 | 116 |
| | | 600 | 676 | 0.14 | - | - |
| Complex 1[d] | 562, 658 | 488 | 674 | 0.19 | ND | ND |
| | | 514 | 674 | 0.32 | ND | ND |
| | | 600 | 674 | 0.39 | ND | ND |
| Complex 2[a] | 490 (13), 554 (9.5) | 466 | 571 | 0.15 | 89 | 81 |
| Complex 3[a] | 545 (9.5), 658 (14.3) | 514 | 679 | 0.08 | 83 | 133 |
| Complex 4[a] | 550 (9.4), 656 (14.2) | 514 | 674 | 0.2 | 96 | 124 |
| Complex 5[a] | 445 (9.5), 754 (14.4) | 520 | 782 | ND | 99 | 226 |
| Complex 6[a] | 556 (9.5), 652 (14.4) | 488 | 674 | 0.23 | 49 | 118 |
| | | 514 | 674 | 0.24 | 50 | 118 |
| | | 600 | 674 | 0.34 | - | - |
| Complex 6[b] | 548 (20.0), 652 (15.0) | 488 | 566 | 0.05 | 43 | 118 |
| | | 514 | 564 | 0.05 | 38 | 116 |
| | | 600 | 668 | 0.23 | - | - |

[a] = in methanol,
[b] = in PBS,
[c] = Complex 1 on streptavidin, d/p = 4
[d] = in glycerine,
[e] = difference between $Em_{max}(A)$ - $Ab_{max}(D)$
ND means not determined.

Table 5

| Complex | Excitation (nm) | Abs Max Fluorochrome #1 | Abs Max Fluorochrome #2 | Abs Max Fluorochrome #3 | Emission (nm) | Quantum Yield (Φ) | Stokes' Shift (nm) | Efficiency of Energy Transfer (%) |
|---|---|---|---|---|---|---|---|---|
| Fluorescein-CY3 $(NH_2)_2$ | 488 | 500 | 558 | - | 574 | 0.041 | 74 | 98.3 |
| Fluorescein-CY3 $(NH_2)_2$-CY5 $(SO_3)_2$ | 488 | 500 | 560 | 650 | 672 | 0.1566 | 172 | 99 |
| Fluorescein-CY3 $(NH_2)_2$-CY7 $(SO_3)_2$ | 488 | 500 | 560 | 754 | 782 | - | 282 | 99 |

Multiparameter analysis can be done of multiple samples to detect the presence of target biological compounds. Each sample is labelled by well known labelling methods with a different complex. For example, one sample suspected of containing a target biological compound is incubated with a single fluorochrome, such as fluorescein, Cascade Blue, a BODIPY dye, or one of the monomethine rigidized dyes, or $CY3O(SO_3)_2$, or $CY3(SO_3)_2$, all emitting in the 500 - 575nm wavelength range (green to orange). A second sample suspected of containing the target biological compound (the same compound or a different compound as that in sample 1), is incubated with a complex of the invention, for example fluorescein-$CY3NH_2$, which will absorb light at 488nm and emits fluorescence at 574nm (orange). Additional samples suspected of containing another target compound are incubated with other labelling complexes of the invention, such as fluorescein-CY3-CY5 and fluorescein-CY3-CY7, both of which light at 488nm, but emit fluorescence at 672nm and 782nm respectively (red to near infra-red). After a suitable period to permit the fluorescent labels to bind with the target compounds, unbound label is removed by washing and the labelled samples are mixed. Detection is possible with a single wavelength excitation source, ie. at laser line 488nm. Each differently labelled sample will fluoresce a different colour at the emission wavelength of its particular label. Those skilled in the art will recognise that the fluorescent labelling complexes of the present invention can be used for a variety of immunofluorescent techniques, including direct and indirect immunoassays, and other known fluorescent detection methods. The conditions of each incubation, eg. pH, temperature and time are known in the art, but generally room temperature is preferred. If reacting with an amine, pH 9.4 is preferred. The pH is adjusted depending on the optimum reaction conditions for the particular reactive groups according to known techniques.

The fluorescent labelling complexes may be used to form reagents by covalently binding the complexes to a carrier material, such as polymer particles, cells, glass beads, antibodies, proteins, enzymes, carbohydrates, lipids and nucleotides or nucleic acids (DNA and RNA) and analogues which have been derivatised to include at least one first reactive group capable of forming a covalent bond with the functional group on the labelling complex (or a functional group capable of forming a covalent bond with a reactive group on the complex, as described above) and at least one second reactive group (or functional group, as the case may be), having specificity for, and being capable of forming a covalent bond with, a target biological compound, such as antibodies, cells, drugs, antigens, bacteria, viruses and other microorganisms. When the carrier has functional groups, it may be antibody or DNA suited for attachment to antigen or a complementary DNA sequence, respectively. When the carrier material has reactive groups on it, the carrier may be a polymer particle or an antigen suitable for attachment to DNA or an antibody for example. Techniques for covalently binding fluorochromes to carrier molecules such as those mentioned are well known in the art and readily available in the literature. The carrier material can further include nucleotides derivatised to contain one of amino, sulphydryl, carboxyl, carbonyl or hydroxyl groups, and oxy or deoxy polynucleic acids derivatised to contain one of amino, thiophosphoryl, sulphydryl, carboxyl, carbonyl or hydroxyl groups. The functional groups on the carrier material which are complementary to. ie. form covalent bonds with, the reactive groups of the labelling complexes of the invention include amino, sulphydryl, carboxyl, carbonyl and hydroxyl groups.

A comparison of the energy transfer complexes of the present invention to the conventional R-phycoerythrin dyes is shown in Table 6 below.

Table 6

| **Complex 2 vs R-Phycoerythrin** | | |
|---|---|---|
| | R-Phycoerythrin | Complex 2 |
| Excitation Wavelength (nm) | 488 | 488 |
| Emission Wavelength (nm) | 580 | 578 |
| 488-laserline Flow-Cytometer | PE fluorescence was greatly reduced at pH 8.5 and extinguished at pH 9.5. | Signals were stable throughout pH range. |
| MW | 240000 | 1667 |
| Staining | Do not penetrate readily into intracellular tissues to reach target antigen. | Labelled antibody penetrates into intracellular tissues to reach target antigen. |
| Binding Rate | Rate of binding to antigen is low. | Rapid binding. |

The energy transfer complexes of the present invention provide a valuable set of fluorescent labels which are particularly useful for multiparameter analysis and importantly, are sufficiently low in molecular weight to permit materials labelled with the fluorescent complexes to penetrate all structures. As such, the complexes are well suited for use as DNA probes. The complexes of the invention and the reagents that can be made from them offer a wide variety of

fluorescent labels with large Stokes' shifts. Those in the art will recognise that the complexes of the invention can be used in a variety of fluorescence applications over a wide range of the visible spectrum.

## Figures

Figure 1 is a schematic illustration of the overlapping absorption and emission spectra of four cyanine fluorochromes that can be used in the energy transfer labelling complexes of the present invention.

Figure 2 illustrates the absorption spectra of two fluorescent labelling complexes, Complex 1 (solid line) in methanol, comprised of one cyanine donor and one cyanine acceptor, and Complex 6 (dotted line) in methanol, comprised of two cyanine donors and one cyanine acceptor.

Figures 3(a) and (b) illustrate the absorbance (solid line) and emission (dotted line) spectra of Complex 1 of the invention made of trimethine and pentamethine cyanine dyes in (a) methanol and (b) PBS.

Figure 4 illustrates the normalised excitation spectra of the Complex 1 in PBS (solid line), methanol (—•••— ), glycerol (— - —), and Complex 1-streptavidin conjugate in PBS (-------).

Figure 5 illustrates the absorbance spectra in PBS of sheep IgG-Complex 1 conjugates at various dye molecule: protein ratios (1 - 4:1) demonstrating that no dimer formation involving either donor or acceptor is evident with increasing dye:protein ratios.

Figure 6 illustrates the two colour flow cytometry analysis of human lymphocytes labelled with anti-CD4-PE and anti-CD3-streptavidin-Complex 1 to mark the helper cell subset of T-cells and total T-cell subset, respectively, showing a subset of Complex 1 labelled cells without the PE signal and a second subset of Complex 1 labelled cells that is PE stained.

## Claims

**1.** A multiparameter method for detecting target biological compounds present in multiple samples using a set of different fluorescent labels including at least two labels, each said label being capable of excitation by a single wavelength excitation source and each label emitting fluorescence at a different wavelength, said method comprising:

a) incubating each separate sample with a different label from said set of fluorescent labels to provide fluorescently-labelled samples;
b) mixing each of said fluorescently-labelled samples to form a mixture containing all samples;
c) irradiating the mixture with said single wavelength excitation source; and
d) detecting the fluorescence emissions corresponding to each of the different fluorescently-labelled samples; **characterised in that** one or more labels of said set of fluorescent labels is a fluorescent labelling complex comprising:

i) a first fluorochrome having first absorption and emission spectra;
ii) a second fluorochrome having second absorption and emission spectra, the wavelength of the emission maximum of said second fluorochrome being longer than the wavelength of the emission maximum of said first fluorochrome, and a portion of the absorption spectrum of said second fluorochrome overlapping a portion of the emission spectrum of said first fluorochrome;
iii) at least one linker for covalently attaching said first and second fluorochromes for transfer of resonance energy between said first and second fluorochromes;
iv) at least one target bonding group capable of forming a covalent bond with said target biological compound;

wherein said target bonding group is a reactive group for reacting with a functional group of the target compounds; and
wherein the combined molecular weight of said first and second fluorochromes and said linker is less than 20,000

Daltons.

2. The method according to claim 1 wherein said set of fluorescent labels comprises a single fluorochrome selected from fluorescein, Cascade blue, a BODIPY dye and a cyanine dye.

3. The method according to claim 1 or claim 2 wherein said first and second fluorochromes in said one or more fluorescent labelling complexes are selected from fluoresceins, pyrene trisulphonates, rhodamines, cyanine dyes and derivatives of the bis-pyrromethine boron difluoride dyes.

4. The method according to any of claims 1 to 3 wherein at least one of said first or second fluorochromes in said one or more fluorescent labelling complexes is a cyanine dye.

5. The method according to any of claims 1 to 4 wherein said fluorescent labelling complexes further comprise water solubilizing constituents attached thereto, said water solubilizing constituents being unreactive with said target bonding group, wherein said water solubilizing constituents are selected from the group consisting of amide, sulphonate, sulphate, phosphate, quaternary ammonium, hydroxyl, guanidinium and phosphonate.

6. The method according to any of claims 1 to 5 wherein said reactive group is selected from the group consisting of succinimidyl ester, isothiocyanate, isocyanate, haloacetamide, dichlorotriazine, maleimide, sulphonyl halide, alkylimidoester, arylimidoester, substituted hydrazine, substituted hydroxylamine, carbodiimide, acyl halide, anhydride, phosphoramidite, acrylate and acrylamide.

7. The method according to claim 1 or claim 2 wherein said fluorescent labelling complex is selected from : Complex 1, $CY3NH_2SO_3$ (Donor) + $CY5(SO_3)_2$ (Acceptor); Complex 2, CY3-$O(SO_3)_2$ (Donor) + $CY3NH_2$ (Acceptor); Complex 3, $CY3NH_2$ (Donor) + CY5COOH (Acceptor); Complex 4, $CY3NH_2$ (Donor) + $CY5(SO_3)_2$ (Acceptor); and Complex 5, $CY3(NH_2)_2$ (Donor) + $CY7(SO_3)_2$ (Acceptor).

8. The method according to claim 1 or claim 2 wherein one or more of said fluorescent labelling complexes further comprises a third fluorochrome having third absorption and emission spectra covalently attached to said second fluorochrome; the wavelength of the emission maximum of said third fluorochrome being longer than the wavelength of the emission maximum of said second fluorochrome and a portion of the emission spectrum of said second fluorochrome overlapping a portion of the absorption spectrum of said third fluorochrome such that excitation of said first fluorochrome produces fluorescence from said third fluorochrome.

9. The method according to any of claims 1 to 8 wherein said labelling complexes are selected from fluorescein-$CY3NH_2$, fluorescein-CY3-CY5 and fluorescein-CY3-CY7.

10. The method according to any of claims 1 to 9 wherein said target biological compounds are selected from the group consisting of antibody, lipid, protein, carbohydrate, nucleotide derivatized to contain one of an amino, sulphydryl, carbonyl, hydroxyl and carboxyl, phosphate and thiophosphate groups and oxy or deoxy polynucleic acids derivatized to contain one of an amino, sulphydryl, carbonyl, hydroxyl and carboxyl, phosphate and thiophosphate groups.

**Patentansprüche**

1. Multiparameterverfahren zum Detektieren von biologischen Zielverbindungen, die in mehreren Proben vorhanden sind, unter Verwenden eines Satzes von unterschiedlichen Fluoreszenzmarkern, die mindestens zwei Marker umfassen, wobei jeder Marker zur Anregung durch eine Anregungsquelle einer einzelnen Wellenlänge fähig ist und jeder Marker Fluoreszenz bei einer unterschiedlichen Wellenlänge emittiert, wobei das Verfahren umfaßt:

    a) Inkubieren jeder getrennten Probe mit einem unterschiedlichen Marker aus dem Satz von Fluoreszenzmarkern, um fluoreszenzmarkierte Proben bereitzustellen;

    b) Mischen jeder der fluoreszenzmarkierten Proben, um eine Mischung zu bilden, die alle Proben enthält;

    c) Bestrahlen der Mischung mit einer Anregungsquelle einer einzelnen Wellenlänge; und

d) Detektieren der Fluoreszenzemissionen entsprechend jeder der unterschiedlichen fluoreszenzmarkierten Proben;

**dadurch gekennzeichnet, daß** ein oder mehrere Marker des Satzes von Fluoreszenzmarkem ein fluoreszenzmarkierender Komplex ist, umfassend:

e) einen ersten Fluoreszenzfarbstoff mit ersten Absorptions- und Emissionsspektren;

ii) einen zweiten Fluoreszenzfarbstoff mit zweiten Absorptions- und Emissionsspektren, wobei die Wellenlänge des Emissionsmaximums des zweiten Fluoreszenzfarbstoffs länger ist als die Wellenlänge des Emissionsmaximums des ersten Fluoreszenzfarbstoffs, und ein Teil des Absorptionsspektrums des zweiten Fluoreszenzfarbstoffs einen Teil des Emissionsspektrums des ersten Fluoreszenzfarbstoffs überlappt;

iii) mindestens einen Linker zum kovalenten Binden des ersten und zweiten Fluoreszenzfarbstoffes zum Transfer von Resonanzenergie zwischen dem ersten und dem zweiten Fluoreszenzfarbstoff;

iv) mindestens eine Zielbindungsgruppe, die zum Bilden einer kovalenten Bindung mit der biologischen Zielverbindung fähig ist;

wobei die Zielbindungsgruppe eine reaktive Gruppe zum Reagieren mit einer funktionellen Gruppe der Zielverbindungen ist; und
wobei das kombinierte Molekulargewicht des ersten und zweiten Fluoreszenzfarbstoffs und des Linkers weniger als 20.000 Dalton beträgt.

**2.** Verfahren nach Anspruch 1, bei dem der Satz von Fluoreszenzmarkern einen einzelnen Fluoreszenzfarbstoff umfaßt, der ausgewählt ist aus Fluorescein, Cascade blue, einem BODIPY-Farbstoff und einem Cyanin-Farbstoff.

**3.** Verfahren nach Anspruch 1 oder 2, bei dem der erste und zweite Fluoreszenzfarbstoff in dem einen oder den mehreren fluoreszenzmarkierenden Komplexen ausgewählt sind aus Fluoresceinen, Pyren-Trisulphonaten, Rhodaminen, Cyanin-Farbstoffen und Derivaten der bis-Pyrromethin-Bor-Difluorid-Farbstoffe.

**4.** Verfahren nach einem der Ansprüche 1 bis 3, bei dem mindestens einer des ersten und zweiten Fluoreszenzfarbstoffes in dem einen oder mehreren fluoreszenzmarkierenden Komplexen ein Cyanin-Farbstoff ist.

**5.** Verfahren nach einem der Ansprüche 1 bis 4, bei dem die fluoreszenzmarkierenden Komplexe weiter daran gebundene wasserlösungsvermittelnde Bestandteile umfassen, wobei die wasserlösungsvermittelnden Bestandteile nicht reaktiv mit der Zielbindungsgruppe sind, und wobei die wasserlösungsvermittelnden Bestandteile aus der Gruppe ausgewählt sind, die besteht aus Amid, Sulphonat, Sulphat, Phosphat, quaternärem Ammonium, Hydroxyl, Guanidinium und Phosphonat.

**6.** Verfahren nach einem der Ansprüche 1 bis 5, bei dem die reaktive Gruppe aus der Gruppe ausgewählt ist, die besteht aus Succinimidyl-Ester, Isothiocyanat, Isocyanat, Haloacetamid, Dichlortriazin, Maleimid, Sulphonyl-Halogenid, Alkylimidoesther, Arylimidoesther, substituiertem Hydrazin, substituiertem Hydroxylamin, Carbodiimid, Acyl-Halogenid, Anhydrid, Phosphoramidit, Acrylat und Acrylamid.

**7.** Verfahren nach Anspruch 1 oder 2, bei dem der fluoreszenzmarkierende Komplex ausgewählt ist aus: Komplex 1, $CY3NH_2SO_3$ (Donor) + $CY5(SO_3)_2$ (Akzeptor); Komplex 2, $CY3\text{-}O(SO_3)_2$ (Donor) + $CY3NH_2$ (Akzeptor); Komplex 3, $CY3NH_2$ (Donor) + CY5COOH (Akzeptor); Komplex 4, $CY3NH_2$ (Donor) + $CY5(SO_3)_2$ (Akzeptor); und Komplex 5, $CY3(NH_2)_2$ (Donor) + $CY7(SO_3)_2$ (Akzeptor).

**8.** Verfahren nach Anspruch 1 oder 2, bei dem einer oder mehrere der fluoreszenzmarkierenden Komplexe ferner einen kovalent an den zweiten Fluoreszenzfarbstoff gebundenen dritten Fluoreszenzfarbstoff mit dritten Absorptions- und Emissionsspektren umfassen, wobei die Wellenlänge des Emissionsmaximums des dritten Fluoreszenzfarbstoffs länger ist als die Wellenlänge des Emissionsmaximums des zweiten Fluoreszenzfarbstoffs, und ein Teil des Emissionsspektrums des zweiten Fluoreszenzfarbstoffs einen Teil des Absorptionsspektrums des dritten Fluoreszenzfarbstoffs derart überlappt, daß die Anregung des ersten Fluoreszenzfarbstoffs eine Fluoreszenz von dem dritten Fluoreszenzfarbstoff erzeugt.

**9.** Verfahren nach einem der Ansprüche 1 bis 8, bei dem die markierenden Komplexe ausgewählt sind aus Fluore-

scein-$CY3NH_2$, Fluorescein-CY3-CY5 und Fluorescein-CY3-CY7.

**10.** Verfahren nach einem der Ansprüche 1 bis 9, bei dem die biologischen Zielverbindungen aus der Gruppe ausgewählt sind, die besteht aus Antikörpern, Lipid, Protein, Kohlenhydrat, Nukleotid, die derivatisiert sind, um eine von einer Amino-, Sulfhydryl-, Carbonyl-, Hydroxyl- und Carboxyl-, Phosphat- und Thiophosphat-Gruppe zu enthalten, und Oxy- oder Desoxypolynukleinsäuren, die derivatisiert sind, um eine von einer Amino-, Sulfhydryl-, Carbonyl-, Hydroxyl- und Carboxyl-, Phosphat- und Thiophosphat-Gruppe zu enthalten.

## Revendications

**1.** Procédé de détection à paramètres multiples de composés biologiques cibles présents dans des échantillons multiples en utilisant une série de différents marqueurs fluorescents comportant au moins deux marqueurs, chacun desdits marqueurs pouvant être excité par une source d'excitation à longueur d'onde unique et chaque marqueur émettant une fluorescence à une longueur d'onde différente, ledit procédé comprenant :

a) l'incubation de chaque échantillon séparé avec un marqueur différent provenant de ladite série de marqueurs fluorescents afin de produire des échantillons marqués de manière fluorescente ;
b) le mélange de chacun desdits échantillons marqués de manière fluorescente afin de former un mélange contenant tous les échantillons ;
c) l'exposition du mélange à ladite source d'excitation à longueur d'onde unique ; et
d) la détection des émissions fluorescentes correspondant à chacun des différents échantillons marqués de manière fluorescente ;

**caractérisé en ce qu'**un ou plusieurs marqueurs de ladite série de marqueurs fluorescents est un complexe de marquage fluorescent comprenant :

i) un premier fluorochrome présentant des premiers spectres d'absorption d'émission ;
ii) un deuxième fluorochrome présentant des deuxièmes spectres d'absorption et d'émission, la longueur d'onde du maximum d'émission dudit deuxième fluorochrome étant supérieure à la longueur d'onde du maximum d'émission dudit premier fluorochrome, et une partie du spectre d'absorption dudit deuxième fluorochrome recouvrant une partie du spectre d'émission dudit premier fluorochrome ;
iii) au moins un élément liant destiné à lier de manière covalente lesdits premier et deuxième fluorochromes afin d'assurer le transfert d'énergie de résonance entre lesdits premier et deuxième fluorochromes ; et
iv) au moins un groupe de liaison cible pouvant former une liaison covalente avec ledit composé biologique cible ;

dans lequel ledit groupe de liaison cible est un groupe réactif destiné à réagir avec un groupe fonctionnel des composés cibles ; et

dans lequel le poids moléculaire combiné desdits premier et deuxième fluorochromes et dudit élément liant est inférieur à 20 000 Daltons.

**2.** Procédé selon la revendication 1, dans lequel ladite série de marqueurs fluorescents comprend un seul fluorochrome sélectionné à partir de la fluorescéine, du bleu cascade, d'un colorant BODIPY et d'un colorant cyanine.

**3.** Procédé selon la revendication 1 ou 2, dans lequel lesdits premier et deuxième fluorochromes dans lesdits un ou plusieurs complexes de marquage fluorescent sont sélectionnés à partir de fluorescéines, de trisulfonates de pyrène, de rhodamines, de colorant cyanine et de dérivés des colorants à base de difluorure de bore bis-pyrrométhine.

**4.** Procédé selon l'une quelconque des revendications 1 à 3, dans lequel au moins l'un desdits premier et deuxième fluorochromes sur lesdits un ou plusieurs complexes de marquage fluorescent est un colorant cyanine.

**5.** Procédé selon l'une quelconque des revendications 1 à 4, dans lequel lesdits complexes de marquage fluorescents comprennent, en outre, des constituants de solubilisation à l'eau fixés dessus, lesdits constituants de solubilisation à l'eau ne réagissant pas avec ledit groupe de liaison cible, dans lequel lesdits constituants de solubilisation à l'eau sont sélectionnés à partir du groupe constitué par un amide, un sulfonate, un sulfate, un phosphate, un ammonium quaternaire, un hydroxyle, du guanidinium et un phosphonate.

**6.** Procédé selon l'une quelconque des revendications 1 à 5, dans lequel ledit groupe réactif est sélectionné à partir du groupe constitué par l'ester de succinimidyle, l'isothiocyanate, l'isocyanate, l'haloacétamide, la dichlorotriazine, la maléimide, l'halide de sulfonyle, l'alkylimidoester, l'arylimidoester, l'hydrazine substituée, l'hydroxylamine substituée, la carbodiimide, l'halide d'acyle, l'anhydride, le phosphoramidite, l'acrylate et l'acrylamide.

**7.** Procédé selon la revendication 1 ou 2, dans lequel ledit complexe de marquage fluorescent est sélectionné à partir de : complexe 1, $CY3NH_2SO_3$ (donneur) + $CY5(SO_3)_2$ (receveur) ; complexe 2, $CY3\text{-}O(SO_3)_2$ (donneur) + $CY3NH_2$ (receveur) ; complexe 3, $CY3NH_2$ (donneur) + CY5COOH (receveur) ; complexe 4, $CY3NH_2$ (donneur) + $CY5(SO_3)_2$ (receveur) ; et complexe 5, $CY3(NH_2)_2$ (donneur) + $CY7(SO_3)_2$ (receveur).

**8.** Procédé selon la revendication 1 ou 2, dans lequel un ou plusieurs desdits complexes de marquage fluorescent comprend, en outre, un troisième fluorochrome, présentant des troisièmes spectres d'absorption et d'émission, lié de manière covalente audit second fluorochrome ; la longueur d'onde du maximum d'émission dudit troisième fluorochrome étant supérieure à la longueur d'onde du maximum d'émission dudit deuxième fluorochrome et une partie du spectre d'émission dudit deuxième fluorochrome recouvrant une partie du spectre d'absorption dudit troisième fluorochrome de telle sorte que l'excitation dudit premier fluorochrome produit une fluorescence à partir dudit troisième fluorochrome.

**9.** Procédé selon l'une quelconque des revendications 1 à 8, dans lequel lesdits complexes de marquage sont sélectionnés à partir de fluorescéine-$CY3NH_2$, fluorescéine-CY3-CY5 et fluorescéine-CY3-CY7.

**10.** Procédé selon l'une quelconque des revendications 1 à 9, dans lequel lesdits composés biologiques cibles sont sélectionnés à partir du groupe constitué par un anticorps, un lipide, une protéine, un carbohydrate, un nucléotide dérivé afin de contenir l'un de groupes amino, sulphydryle, carbonyle, hydroxyle et carboxyle, phosphates et thiophosphates et des acides oxy- ou désoxy-polynucléiques dérivés afin de contenir l'un de groupes amino, sulphydryle, carbonyle, hydroxyle et carboxyle, phosphate et thiophosphate.

**Figure 1**

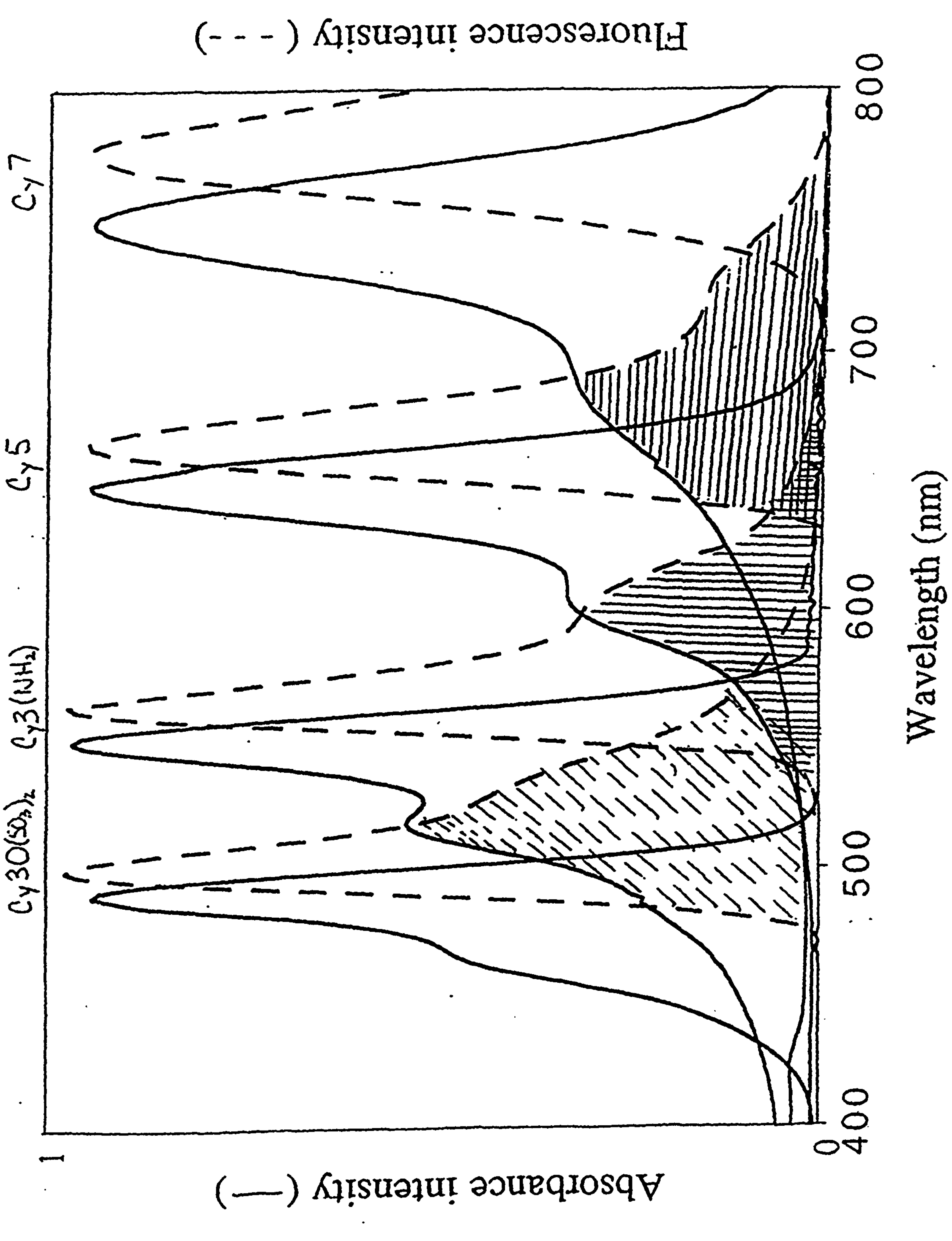
Fluorescence intensity ( - - - )
Absorbance intensity (——)
Wavelength (nm)
400
500
600
700
800
0
1
Cy3O(SO3)2
Cy3(NH2)
Cy5
Cy7

**Figure 2**

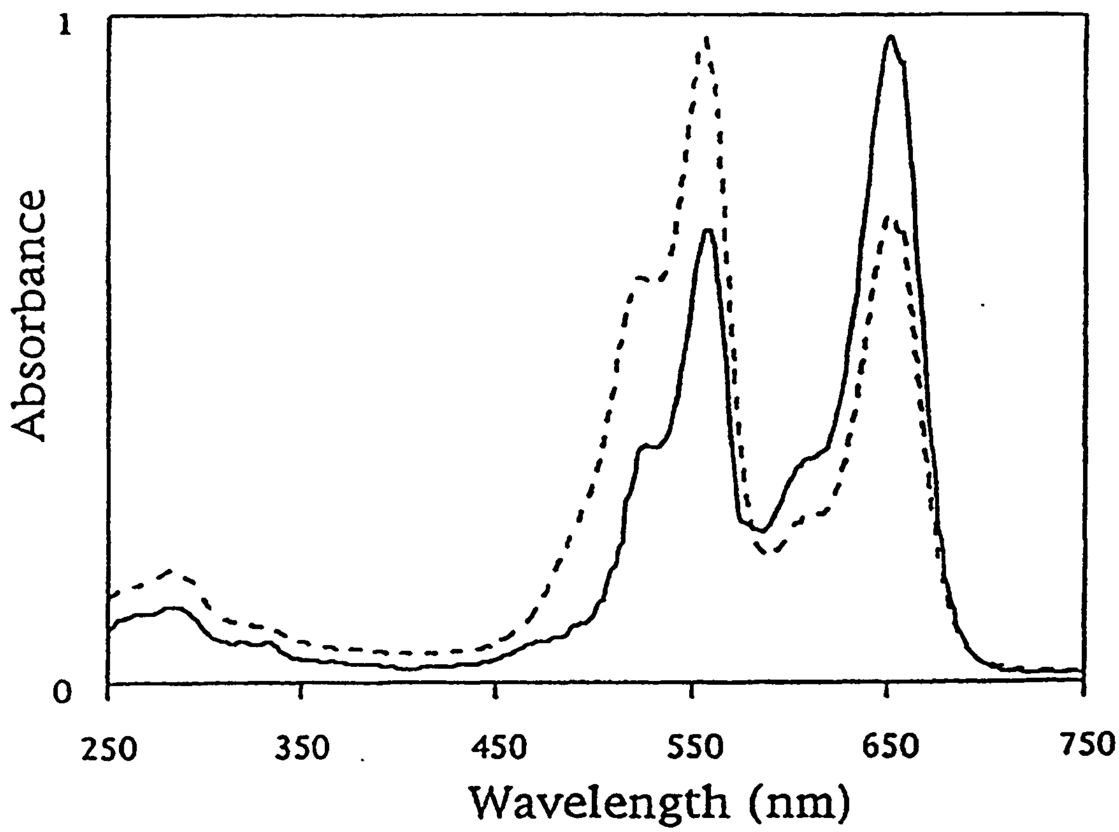

**Figure 3**

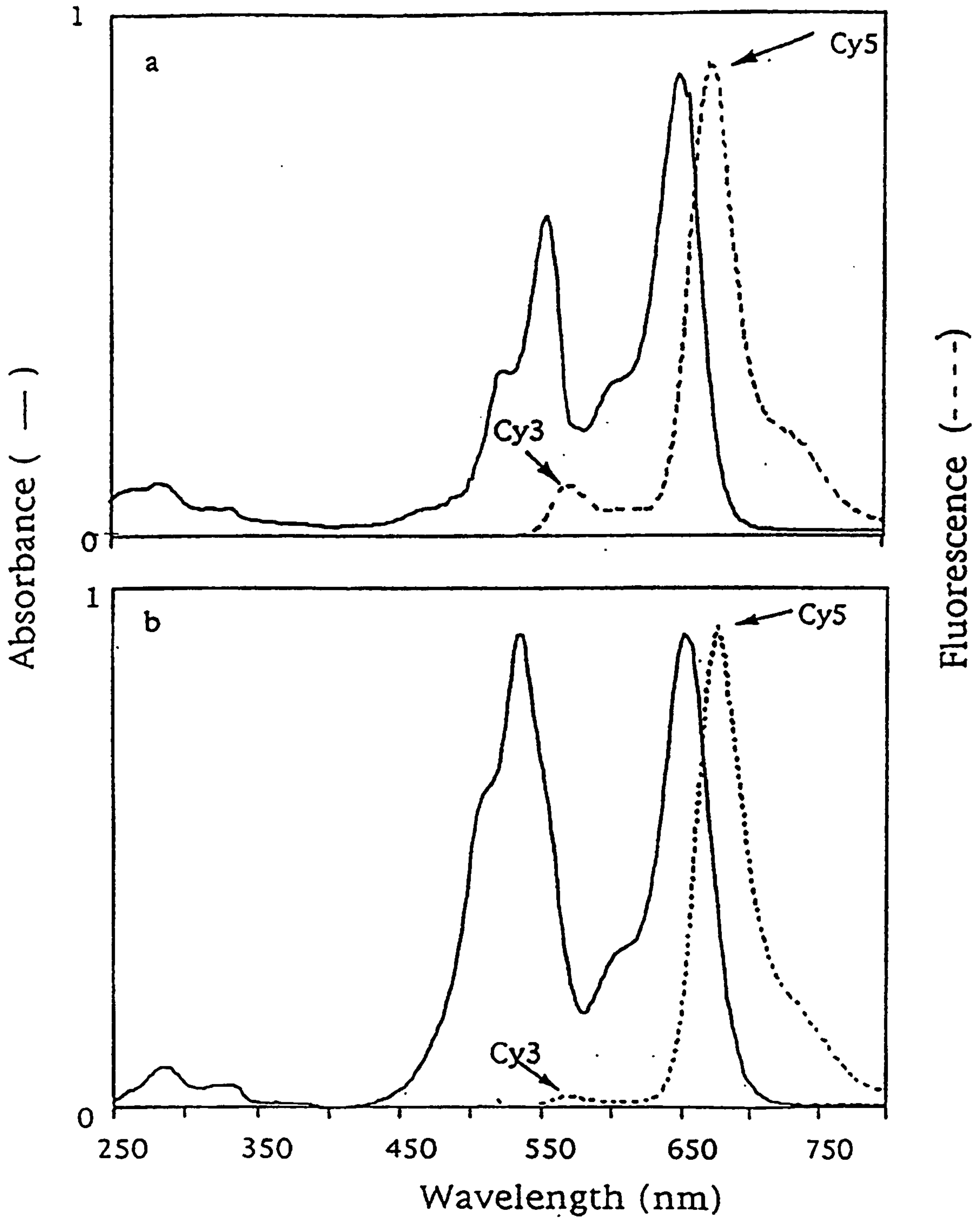
1
a
Cy5
Cy3
0
1
b
Cy5
Cy3
0
250
350
450
550
650
750
Wavelength (nm)
Absorbance ( — )
Fluorescence ( - - - )

**Figure 4**

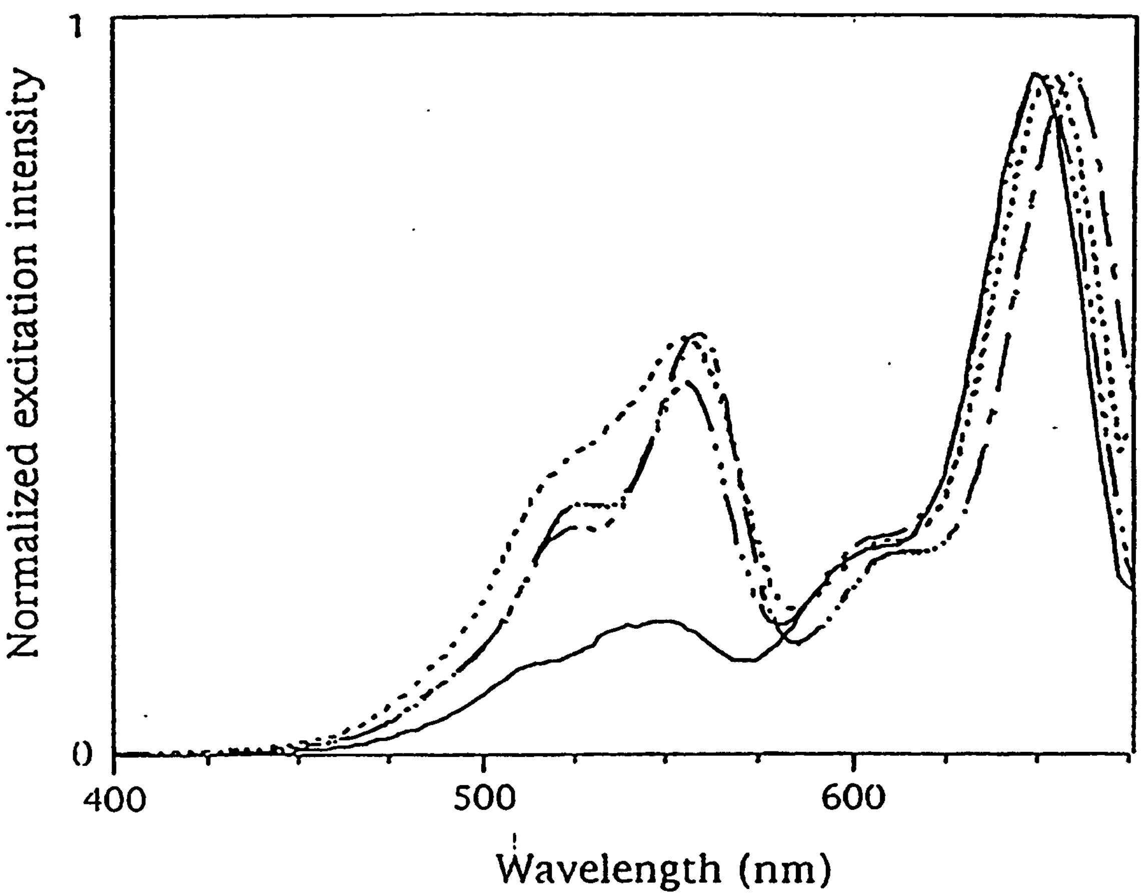
1
0
400
500
600
Normalized excitation intensity
Wavelength (nm)

Figure 5

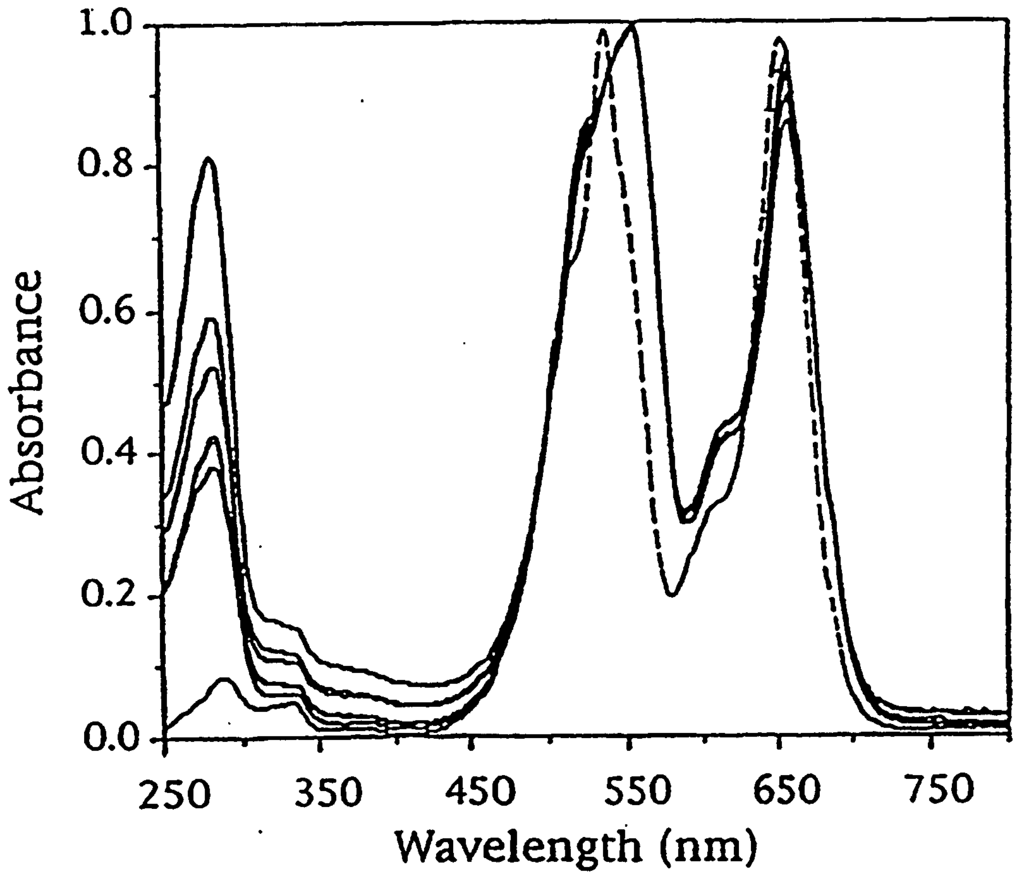
1.0
0.8
0.6
0.4
0.2
0.0
Absorbance
250
350
450
550
650
750
Wavelength (nm)

**Figure 6**

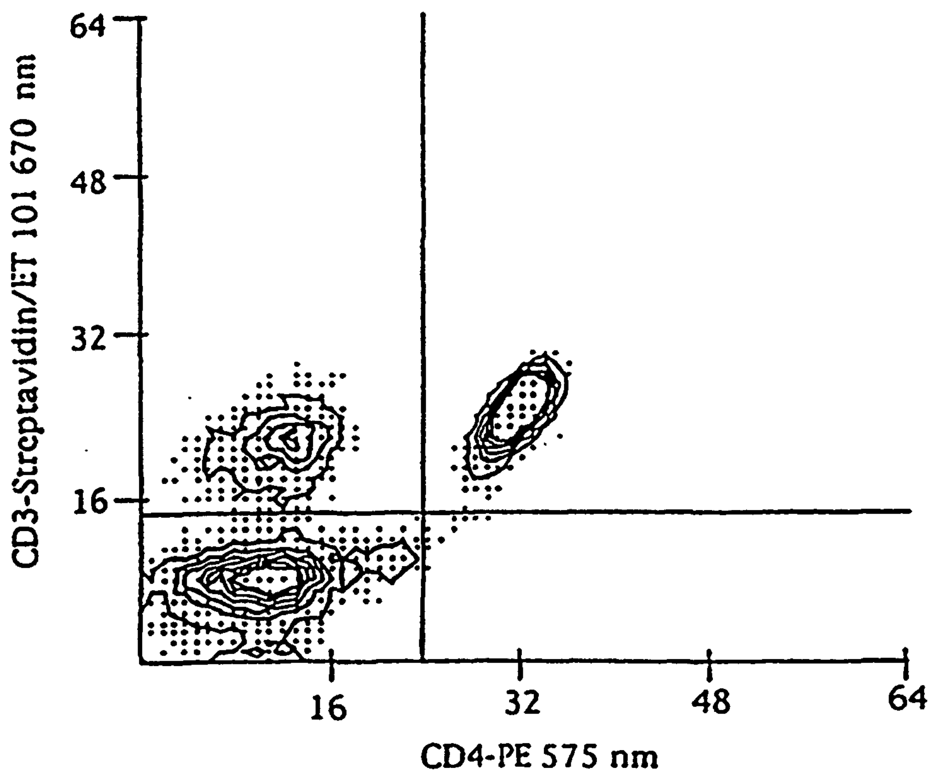
64
48
32
16
CD3-Streptavidin/ET 101 670 nm
16
32
48
64
CD4-PE 575 nm